# FASCICULE DE BREVET EUROPEEN

(11) **EP 0 628 534 B1**
(45) Date de publication et mention de la délivrance du brevet: **29.10.1997**
(21) Numéro de dépôt: 94401057.8
(22) Date de dépôt: 11.05.1994
(51) Int. Cl.: C07C 59/42, C07C 51/00

(54) **Procédé de synthèse stéréospécifique de leucotriène B4 sous sa configuration 6Z, 8E, 1OE et produits intermédiaires**
Verfahren für stereospezifische Synthese von Leucotrien B4 mit Konfiguration 6Z, 8E, 10E und Zwischenprodukte
Process for the stereospecific synthesis of leucotriene B4 having configuration 6Z, 8E, 10E and intermediate products

(30) Priorité: 01.06.1993 FR 9306500
(43) Date de publication de la demande: 14.12.1994
(73) Titulaire: L'OREAL, 75008 Paris (FR)
(72) Inventeur: Solladie, Guy, F-67000 Strasbourg (FR); Stone, Guy, Ch-4123 Allschwill (CH); Urbano-Pujol, Antonin, E-28043 Madrid (ES); Maignan, Jean, F-93290 Tremblay Les Gonesse (FR)
(74) Mandataire: Peuscet, Jacques

(56) Documents cités:
- EP-A- 0 580 476
- THE JOURNAL OF EXPERIMENTAL MEDICINE, vol.154, no.4, 4 Octobre 1981, DEP.MED.,HARVARD MED.SCH.,BOSTON,USA pages 1243 - 1248 R.A.LEWIS ET AL. 'FUNCTIONAL CHARACTERIZATION OF SYNTHETIC LEUKOTRIENE B AND ITS STEREOCHEMICAL ISOMERS.'

## Description

La présente invention concerne un procédé de synthèse stéréospécifique de leucotriène B₄ (ci-après désigné par LTB₄), sous sa configuration 6Z, 8E, 10E (ou 6-cis, 8-trans, 10-trans) et certains produits intermédiaires obtenus au cours de ce procédé.

On sait que les leucotriènes sont des métabolites de l'acide arachidonique dans les leucocytes. L'acide arachidonique est transformé en un composé époxyde instable dénommé leucotriène A₄, qui est lui-même transformé par la 5-lipoxygénase en LTB₄. Le LTB₄ provoque l'adhésion et le mouvement chemotactique des leucocytes et stimule l'aggrégation, la libération d'enzymes et la formation de superoxydes dans les neutrophiles. Le LTB₄ est donc impliqué dans les processus inflammatoires et allergiques.

Le LTB₄ peut difficilement être isolé en quantités suffisantes à partir de ses sources biologiques et il est de plus en plus demandé pour des études pharmacologiques, en particulier dans le cadre de recherches biologiques sur les processus inflammatoires. Dans ces recherches, il est extrêmement important d'obtenir le leucotriène sous une forme stéréospécifique bien déterminée.

La présente invention concerne un procédé de synthèse stéréospécifique du LTB₄ sous sa forme 6Z, 8E, 10E, qui a pour formule : 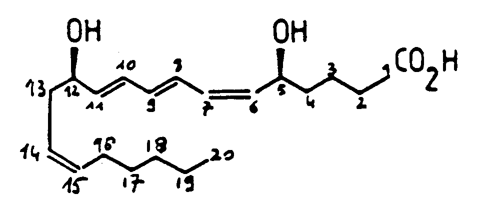 Ce leucotriène a donc dans sa partie triénique centrale une stéréochimie cis, trans, trans.

On a proposé de nombreux procédés de synthèse de LTB₄ ayant cette structure, en particulier par des réactions de Wittig. De tels procédés sont décrits, par exemple, dans les documents suivants :

E.J. Corey, A. Marfat, G. Goto, F. Brion JACS 1980, 102, 7984 ; E.J. Corey, A. Marfat, J. Munroe, K.S. Kim, P.B. Mopkius, F. Brion, Tetrahedron Lett. 1981, 22, 1077 ; Y. Guindon, R. Zamboni, C. Lau, J. Rokach, Tetrahedron Lett. 1982, 23, 739 ; R. Zamboni, J. Rokach, Tetrahedron Lett. 1982, 23, 2631 ; L.J. Mills, P.C. North, Tetrahedron Lett. 1983, 24, 409 ; K.C. Nicolson, R.E. Zipkin, R.E. Dolle, B.D. Harris, JACS 1984, 106, 3548 ; C.K. Hen, D. Di Tullio, Y.F. Wang, C.J. Sih, J. Org. Chem 1986, 51, 1253 ;Y. Le Merrer, C. Gravier, D. Languin-Micas, J.C. Depezay, Tetrahedron Lett. 1986 27, 4161 ; Y. Kobayashi, T. Shimazaki, F. Seto , Tetrahedron Lett. 1987, 47, 5849 ; Y. Guindon, D. Delorme, C.K. Len, R. Zamboni, J. Org. Chem 1988, 53, 267 ; Y. Le Merrer, C. Granier-Pelletier, D. Micas-Languin, M. Mestre, A. Dureault, J.C. Depezay, J. Org. Chem 1989, 54, 2409 ; M. Avignon-Tropis, M. Treilhov, J. Lebreton, J.R. Pougny, I. Frechard-Ortuno, C. Huynh, G. Linstrumelle, Tetrahedron Lett. 1989, 30, 6335 ; Y. Kobayashi, T. Shimazaki, H. Taguchi, F. Seto, J. Org. Chem 1990, 55, 5324 ; M. Avignon-Tropis, M. Treilhov, J.R. Pougny, I. Frechard-Ortuno, G. Linstrumelle, Tetrahedron Lett. 1991, 35, 7279 ; M. Avignon-Tropis, J.M. Berjeaud, J.R. Pougny, I. Frechard-Ortuno, D. Guillerm, G. Linstrumelle, J. Org. Chem 1992, 57, 651 ; M. Treilhov, A. Fauve, J.R. Pougny, J.C. Promé, M. Veschambre, J. Org. Chem 1992, 57, 3203.

On a également proposé dans EP-A-0 580 476 un procédé de fabrication du LTB₄ sous une autre forme stéréochimique : 6-trans.

Cependant, ces procédés ne permettent pas d'obtenir, avec un bon rendement et une bonne stéréospécificité, le LTB₄ ayant la configuration cis, trans, trans désirée.

La présente invention concerne un procédé, qui permet d'obtenir le LTB₄ désiré avec un meilleur rendement et une bonne stéréospécificité.

La présente invention a donc pour objet un procédé de synthèse stéréospécifique de LTB₄ de configuration 6Z, 8E, 10E ayant pour formule 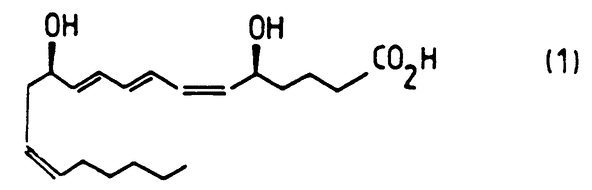 caractérisé par le fait que :
1) on prépare un dibenzoate de formule (2) : 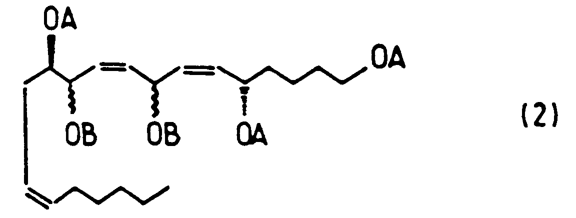 formule (2) dans laquelle A représente un radical silyle, en particulier tertiobutyldiméthylsilyle (ci-après désigné par TBDMS) ou tertiobutyldiphénylsilyle (ci-après désigné par TBDPS), les trois groupes A pouvant être des radicaux silyle différents l'un de l'autre et B représente un radical benzoyle non substitué ou benzoyle substitué par un radical alkyle ou alcoxy en C₁-C₆, de préférence un radical benzoyle non substitué (ci-après désigné par Bz) ;
2) on fait subir au dibenzoate de formule (2) une élimination réductrice contrôlée à l'aide d'un amalgame de sodium à une température comprise entre -10°C et -30°C, en présence d'un solvant, de façon à obtenir un triéther de formule (3): 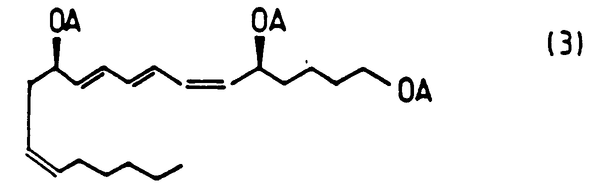 dont on transforme, ensuite, les groupes -OA en positions 5 et 12 en groupe -OH et le groupe -CH₂OA en position 1 en groupe -COOH pour obtenir le LTB₄ de formule (1).

Comme solvant, on utilise, de préférence, le tétrahydrofuranne, le méthanol ou leurs mélanges.

Dans la présente description et dans les revendications, les radicaux A et B correspondent toujours aux définitions qui viennent d'en être fournies.

De préférence, dans le dibenzoate de formule (2), les fonctions alcool en positions 1 et 5 sont substituées ou bloquées par des radicaux tertiobutyldiméthylsilyle alors que l'alcool en position 12 est substitué par un radical tertiobutyldiphénylsilyle. Le radical B est, de préférence, un radical benzoyle non substitué (Bz). Le composé préféré est donc le dibenzoate de formule (2'):

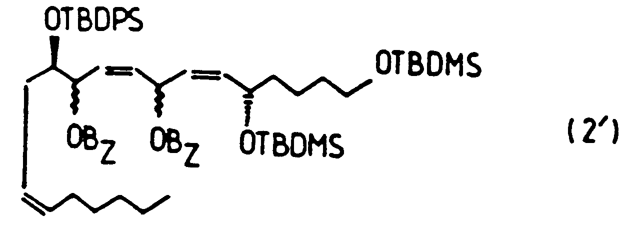

Selon l'invention, on a donc trouvé qu'il est possible de former un diène tout trans à partir d'un dibenzoate allylique tout en conservant la stéréochimie de la double liaison voisine.

Il était connu dans la synthèse de la vitamine A et du 13-cis rétinol (G. Solladié, A. Girardin, G. Lang, J. Org. Chem. 1989, 54, 2620) de réaliser la réaction de formation d'un diène tout trans à partir d'un diol allylique avec le titane basse valence [Ti(O)] : 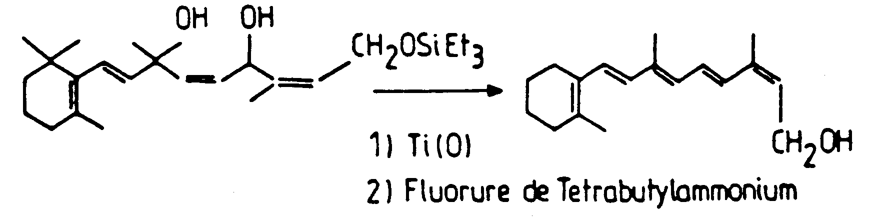 Et = Ethyle

On pouvait donc penser que cette réaction serait possible lorsque l'on traite l'alcool précurseur du dibenzoate de formule (2) dont la configuration centrale s'écrit : 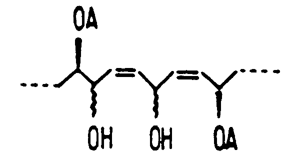 mais il s'est avéré que, dans le cas de la présence d'un alcool secondaire allylique d'un côté et d'un alcool secondaire bis-allylique de l'autre, la réduction par [Ti(O)] ne donne pas de bons résultats. En revanche, on a trouvé, selon l'invention et de façon imprévisible et surprenante, qu'en protégeant les deux alcools sous forme de dibenzoate et en utilisant l'amalgame de sodium au lieu du [Ti(O)] pour le transfert monoélectronique, la réaction était tout à fait possible dans d'excellentes conditions, le rendement en triène cis, trans, trans, étant quantitatif. La réaction peut être schématisée de la façon suivante :

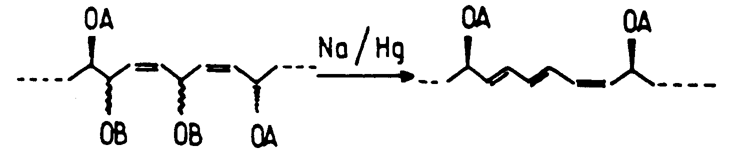

Selon l'invention, le triéther triénique de formule (3) est, de préférence, transformé en LTB₄ de formule (1) de la façon suivante :
1) dans un premier stade, on fait réagir le triéther de formule (3) avec le dichromate de pyridinium à température ambiante en présence d'un solvant, de préférence le diméthylformamide, de façon à obtenir l'acide de formule (4) : 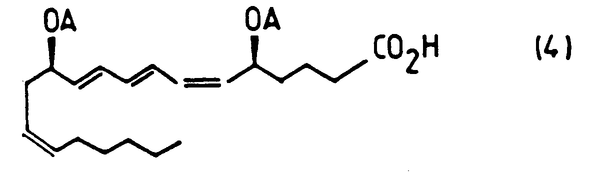 et
2) dans un second stade, on traite l'acide de formule (4) par une solution de fluorure de tétrabutylammonium dans un solvant, de préférence le tétrahydrofuranne, pour obtenir le LTB₄ de formule (1).

En effet, on a également trouvé, de façon surprenante, qu'il était possible de couper l'éther silylé en bout de chaîne et de l'oxyder par le dichromate de pyridinium, sans toucher aux deux autres éthers silylés en positions 5 et 12.

De préférence, le dibenzoate de formule (2) est préparé par le procédé suivant:
1) dans une première étape, on fait réagir un alcool propargylique de formule (5): 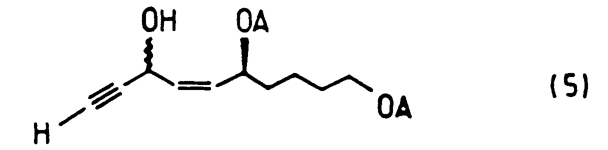 et un aldéhyde de formule (6): 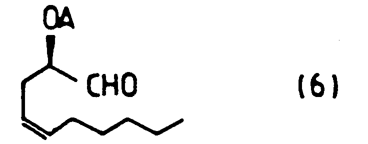 l'alcool propargylique de formule (5) étant préalablement traité par le n-butyllithium, à une température comprise entre -70°C et -90°C, dans un solvant, de préférence le tétrahydrofuranne, puis réagissant avec l'aldéhyde de formule (6) dissous dans un solvant, de préférence le tétrahydrofuranne, la température étant progressivement ramenée à la température ambiante, de façon à obtenir le diol propargylique de formule (7): 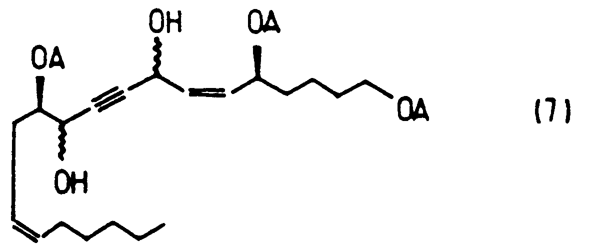
2) dans une deuxième étape, on réduit le diol propargylique de formule (7) en solution dans un solvant, de préférence l'hexane, avec le catalyseur de Lindlar en présence de quinoléine à température ambiante de façon à obtenir le diol de formule (8) : 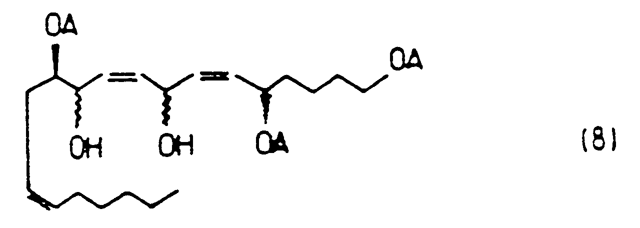 et
3) dans une troisième étape, le diol de formule (8) est traité par le chlorure de benzoyle en solution dans un solvant, de préférence la pyridine, à température ambiante, de façon à obtenir le dibenzoate de formule (2) défini ci-dessus.

L'aldéhyde de formule (6) défini ci-dessus est un composé connu qui a, par exemple, été décrit dans G. Solladié, C. Hamdouchi, C. Ziani-Charif- Tétrahedron Asymmetry, 1991 2, 457.

Le catalyseur de Lindlar est constitué de Pd/CaCO₃/PbO; il est, par exemple, décrit dans "Fieser and Fieser" "Reagents for organic synthesis" - J. Wiley, 1967 - Vol. 1 page 566.

On prépare, de préférence, l'alcool propargylique de formule (5) : 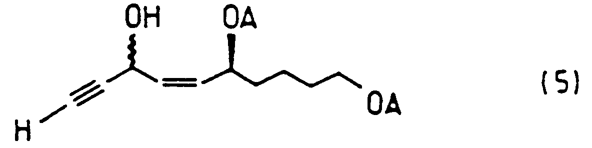 par le procédé suivant:
1) dans une première étape, on utilise, comme produit de départ, l'aldéhyde de formule (9): 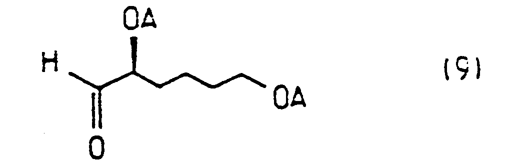 ce composé ayant été décrit, pour A = TBDMS à la 8ème étape de l'exemple de la demande de brevet français 92-08978 déposée par la demanderesse le 21 Juillet 1992, et on le fait réagir avec de la triphénylphosphine et du zinc en poudre dans un solvant, de préférence le dichlorométhane, puis avec CBr₄, à température ambiante, de façon à obtenir le dibromure de formule (10): 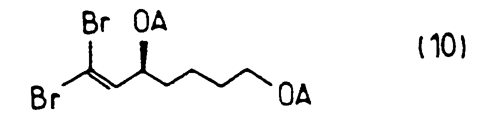
2) dans une deuxième étape, on fait ensuite réagir le dibromure de formule (10) avec du n-butyllithium à une température inférieure à - 50°C, de préférence de l'ordre de -80°C, dans un solvant, de préférence le tétrahydrofuranne, pour obtenir l'alcyne de formule (11): 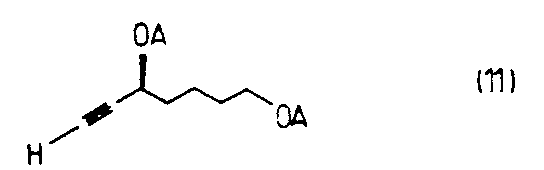
3) dans une troisième étape, on fait réagir l'alcyne de formule (11) en solution dans un solvant, de préférence le tétrahydrofuranne, avec une solution de n-butyllithium à une température comprise entre - 70°C et -90°C puis avec une solution de N-formylpiperidine, la température étant ramenée progressivement à la température ambiante, de façon à obtenir l'aldéhyde propargylique de formule (12): 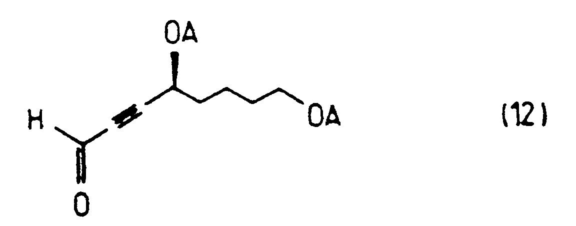
4) dans une quatrième étape, on met l'aldéhyde de formule (12) en présence du catalyseur de Lindlar et de quinoléine en milieu solvant, de préférence le tétrahydrofuranne, sous atmosphère d'hydrogène, de façon à obtenir l'aldéhyde de formule (13): 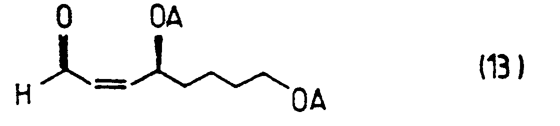 et
5) dans une cinquième étape, on dissout l'aldéhyde de formule (13) dans un solvant, en particulier le tétrahydrofuranne, puis on ajoute du bromure d'acétylène-magnésium de façon à obtenir l'alcool propargylique de formule (5).

Dans toute la présente description et dans les revendications, le terme "température ambiante" désigne une température dans la gamme de celles susceptibles d'être atteintes dans un local fermé non climatisé en climat tempéré, c'est-à-dire une température approximativement comprise entre 5 et 35°C.

Plusieurs des composés préparés comme produits intermédiaires au cours du processus de préparation ci-dessus défini sont nouveaux. C'est le cas des composés de formule 2 à 5, 7, 8 et 10 à 13.

Par conséquent, la présente invention a également pour objet, à titre de composés chimiques nouveaux utiles comme produits intermédiaires, les composés suivants :
a) le dibenzoate de formule 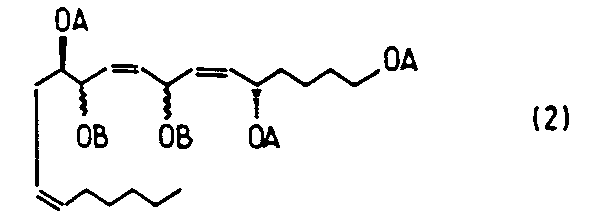 de préférence, le dibenzoate de formule 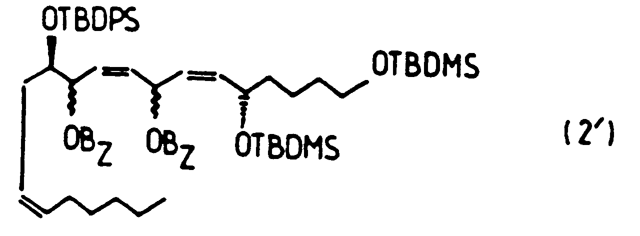
b) le triène de formule 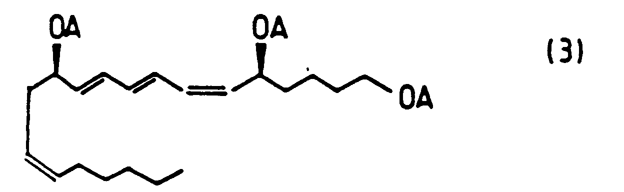 de préférence, le triène de formule 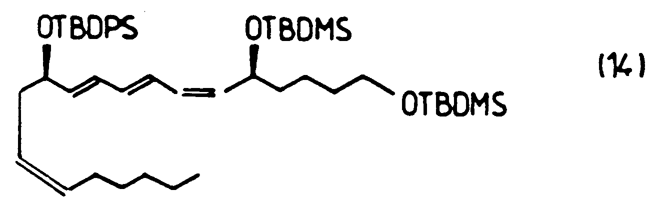
c) l'acide de formule 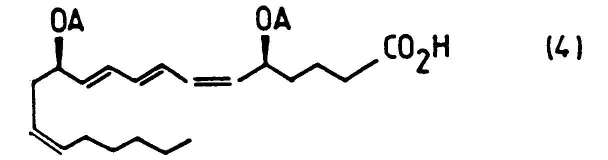 de préférence, l'acide de formule 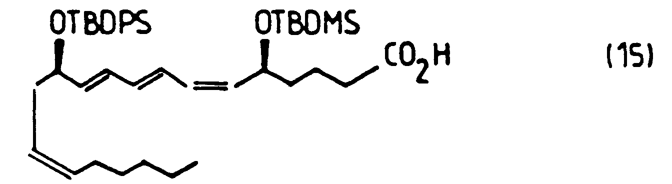
d) l'alcool propargylique de formule 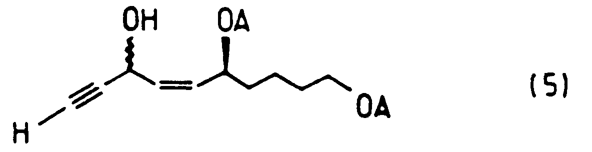 de préférence, l'alcool propargylique de formule 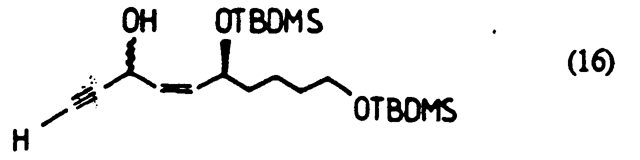
e) le diol propargylique de formule 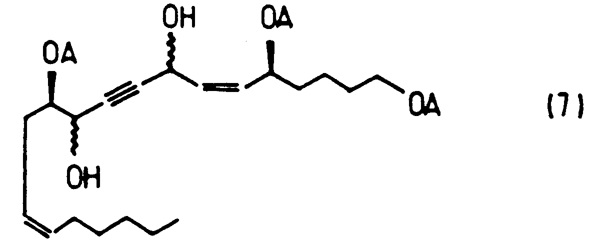 de préférence le diol propargylique de formule 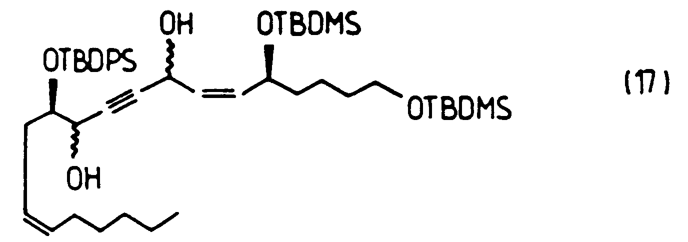
f) le diol de formule 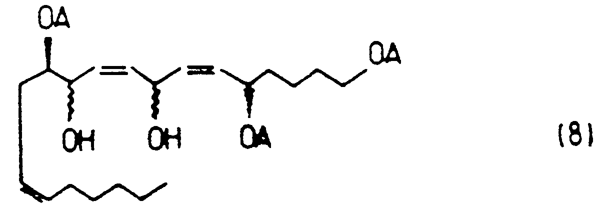 de préférence, le diol de formule 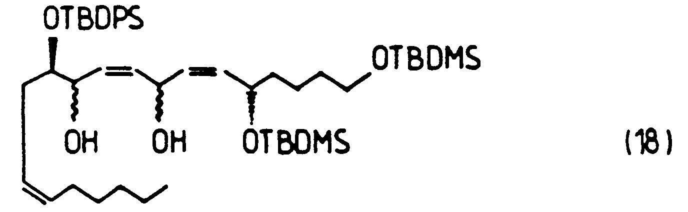
g) l'aldéhyde de formule 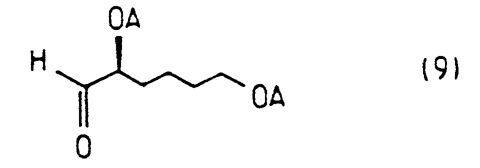 où l'un au moins des deux groupes A représente TBDPS
h) le dibromure de formule 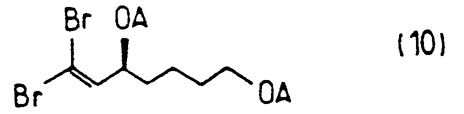 de préférence, le dibromure de formule 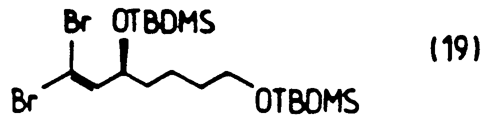
i) l'alcyne de formule 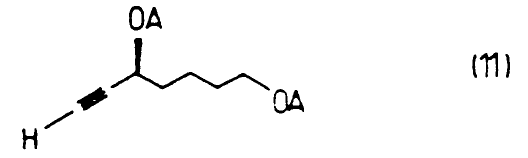 de préférence, l'alcyne de formule 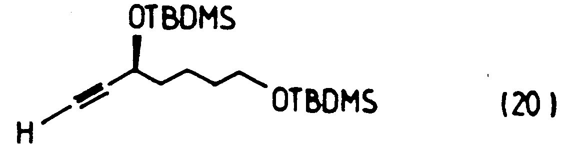
j) l'aldéhyde propargylique de formule 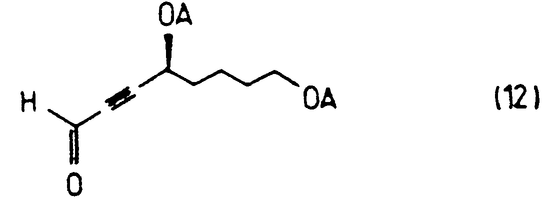 de préférence, l'aldéhyde propargylique de formule 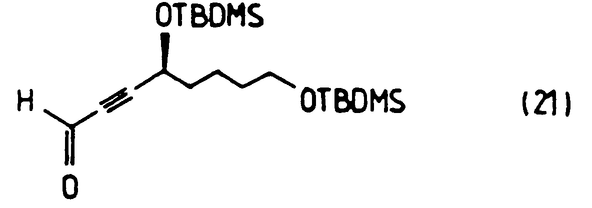
k) l'aldéhyde de formule 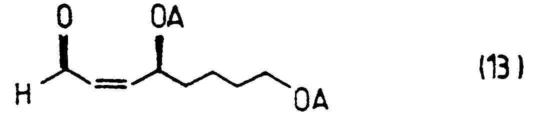 de préférence, l'aldéhyde de formule

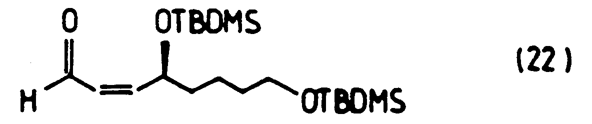

Un exemple de préparation est donné ci-après, à titre purement illustratif et non limitatif.

### EXEMPLE

### 1ère étape : Préparation de (S)-3,7-bis(t-butyldiméthylsilyloxy)-1,1-dibromo heptène de formule

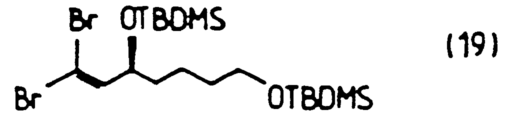

On ajoute une solution de triphénylphosphine (14,5 g, 55 mmol., 10 équivalents) dans CH₂Cl₂ (70 ml) sur du zinc en poudre (3,6 g, 55 mmol., 10 équivalents). On ajoute ensuite une solution de CBr₄ (18,25 g, 55 mmol., 10 équivalents) dans CH₂Cl₂ (80 ml). On agite ensuite le mélange réactionnel pendant 24 heures à température ambiante et on ajoute le (-)2(S)-2,6-bis(t-butyldiméthylsilyloxy)-hexan-1-al (2,0 g, 55 mmol.) en solution dans CH₂Cl₂ (50 ml). Après agitation pendant 2 heures, on ajoute de l'hexane et on filtre les solides. On lave le solide à l'hexane, on évapore les solvants. Le brut de la réaction est ensuite chromatographié sur silice (éther/hexane : 1/100). On obtient 2,6 g de dibromure (19) pur (91 %).

Les caractéristiques du produit obtenu sont les suivantes :
a) [α]_{D} = -2,5 (c = 1, CHCl₃)
b) ¹H RMN (200 MHz, CDCl₃) : δ: 6,37 (d, 1H, J = 8,1 Hz, H-2), 4,27 (m, 1H, H-3), 3,61 (t, 2H, J = 6,1 Hz, H-7), 1,6-1,3 (m, 6H, H-4, H-5, H-6), 0,89 et 0,88 (2s, 18 H, t-Bu), 0,08, 0,06 et 0,05 (3s, 12 H, CH₃-Si)
c) ¹³C RMN (CDCl₃) : δ : 142,2 (C-2), 88 (C-1), 73,5 (C-3), 63 (C-7), 36,6 et 32,6 (C-4, C-5), 26 et 25,8 (t-Bu), 21,3 (C-6), 18,4 et 18,1 (C-Si), -4,5, -4,9 et -5,3 (CH₃-Si).

### 2ème étape : Préparation de l'alcyne de formule

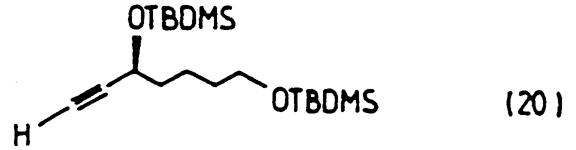

Le dibromure (19) (2,6 g, 5 mmol.) est dissous dans le tétrahydrofuranne (THF) (70 ml) et refroidi à -78°C. On ajoute ensuite goutte à goutte une solution 1,45 M de n-butyllithium dans l'hexane (8,6 ml, 12,5 mmol., 2,5 équivalents). La réaction est maintenue sous agitation pendant 1 heure à -78°C et pendant 1 heure à température ambiante. On dilue avec de l'éther et on hydrolyse avec une solution saturée de NH₄Cl puis on lave avec une solution saturée de NaCl. Après extraction à l'éther, le produit est purifié sur colonne de silice (éther/hexane : 1/50) pour conduire à 1,57 g (88%) de l'alcyne (20).

Les caractéristiques du produit obtenu sont les suivantes :
a) [α]_{D} = -27 (c = 1, CHCl₃)
b) ¹H RMN (200 MHz, CDCl₃) : δ : 4,33 (dt, 1H, J = 2,1 et 6,2 Hz, H-3), 3,61 (t, 2H, J = 6,1 Hz, H-7), 2,36 (d, 1H, J = 2,1 Hz, H-1), 1,7-1,4 (m, 6H, H-4, H-5, H-6), 0,90 et 0,89 (2s, 18H, t-Bu), 0,13, 0,10 et 0,04 (3s, 12H, CH₃Si),
c) ¹³C RMN (CDCl₃) : 85,6 (C-1), 71,9 (C-2), 63 (C-3), 62,7 (C-7), 38,4 et 32,4 (C-4 et C-5), 26 et 25,7 (t-Bu), 21,6 (C-6), 18,3 et 18,2 (C-Si), -4,57, -5,11 et -5,32 (CH₃-Si).

### 3ème étape :Préparation de (-)4(S)-4,8-bis(t-butyldiméthylsilyloxy)-2-octyn-1-al de formule

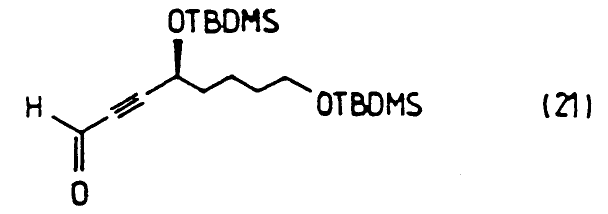

A l'alcyne (20) (1 g, 2,8 mmol.) en solution dans le THF (5 ml) est ajoutée, à -78°C, une solution de 1,5 M de butyllithium dans l'hexane (3 ml, 4,5 mmol., 1,6 équivalent). Après l'addition, la température est remontée à -40°C pendant 1 à 2 heures. On ajoute ensuite, à cette température, une solution de N-formylpiperidine (1 g, 8,8 mmol., 3,1 équivalents) dans le THF (5 ml). L'agitation est poursuivie pendant une heure à -40°C et pendant une heure à température ambiante. Après dilution à l'éther, hydrolyse par une solution saturée de NH₄Cl, lavage des phases organiques avec une solution saturée de NaCl et évaporation des solvants, le produit est purifié par chromatographie (éther/hexane : 5/95). On obtient 0,9 g (83 %) de l'aldéhyde (21).

Les caractéristiques du produit obtenu sont les suivantes :
a) [α]_{D} -31 (c = 1, CHCl₃)
b) ¹H RMN (CDCl₃, 200 MHz) : δ : 9,22 (s, 1H, H-1), 4,52 (t, 1H, J = 6,3 Hz, H-4), 3,61 (t, 2H, J = 6,0 Hz, H-8), 1,8-1,4 (m, 6H, H-5, H-6, H-7), 0,90 et 0,88 (2s, 18H, t-Bu), 0,13, 0,11 et 0,04 (3s, 12H, CH₃Si),
c) ¹³C RMN (CDCl₃) : δ : 176,1 (C-1), 97,5 et 83,5 (C-2, C-3), 62,6 (C-4, C-8), 37,5 et 32,2 (C-5, C-6), 25,8 et 25,6 (t-Bu), 21,4 (C-7), 18,2 et 18 (C-Si), -4,7, -5,2 et -5,4 (CH₃-Si).

### 4ème étape : Préparation de (+) 4(S)-4,8-bis (t-butyldiméthylsilyloxy)-2(Z)-octen-1 al de formule

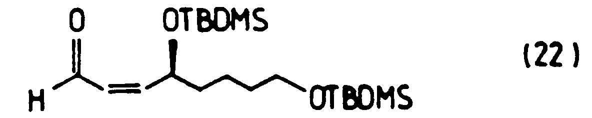

L'aldéhyde propargylique (21) (700 mg, 1,82 mmol.), est dissous dans le THF (70 ml). On ajoute le catalyseur de Lindlar (100 mg) et la quinoléine (100 µl). L'hydrogénation sous atmosphère d'hydrogène est poursuivie pendant une heure. Après dilution à l'éther, filtration sur silice et évaporation des solvants, le produit est purifié par chromatographie (éther/hexane : 5/2). On isole 580 mg (82 %) de l'aldéhyde de formule (22).

Les caractéristiques du produit obtenu sont les suivantes :
a) [α]_{D} = + 9 (c = 1, CHCl₃)
b) ¹H RMN : δ : 10,09 (d, 1H, J = 7,6 Hz, H-1), 6,48 (dd, 1H, J = 8,3 et 11,5 Hz, H-3), 5,89 (ddd, 1H, J = 1,1, 7,6 et 11, 5 Hz, H-2), 4,95 (m, 1H, H-4), 3,59 (t, 2H, J = 6, 1 Hz, H-8), 1,7-1,3 (m, 6H, H-5, H-6 et H-7), 0,87 et 0,86 (2s, 18H, t-Bu), 0,05, 0,02 et 0,01 (3s, 12H, CH₃-Si),
c) ¹³C RMN (CDCl₃) : δ : 191 (C-1), 154,5 et 128 (C-2 et C-3), 68,9 (C-4), 62,8 (C-8), 38 et 32,6 (C-5 et C-6), 25,9 et 25,7 (t-Bu), 21,5 (C-7), 18,3 et 18,1 (C-Si), -4,5, -4,8 et -5,4 (CH₃-Si).

### 5ème étape : Préparation de 6(S)-6,10-bis(t-butyldiméthylsilyloxy)-3-hydroxy-4(Z)-decen-1-yne de formule

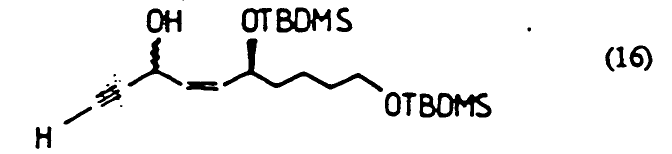

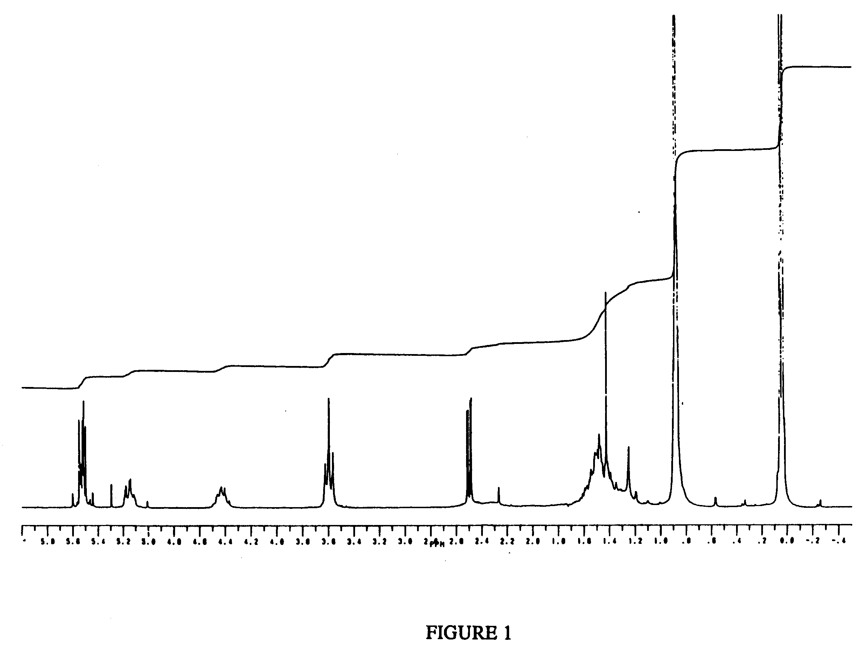

A l'aldéhyde (22) (580 mg, 1,5 mmol.) en solution dans le THF (5 ml) est ajoutée, goutte à goutte, une solution 0,5 M de bromure d'acétylènemagnésium dans le THF (6 ml, 3 mmol., 2 équivalents). Après 15 minutes, on hydrolyse le mélange réactionnel avec une solution saturée de NH₄Cl, on extrait à l'éther, on lave les phases organiques par une solution saturée de NaCl. Après évaporation des solvants et purification par chromatographie, on isole 600 mg (97 %) d'alcool propargylique (16) (mélange de diastéréoisomères). La figure 1 représente le spectre RMN du proton (CDCl₃, 200 M Hz).
([α]_{D} = +11 (C = 0,65, CHCl₃)).

### 6ème étape : Préparation de [14(Z), 5(S), 12(R)]-1,5-bis (t-butyldiméthylsilyloxy)-14-(t-butyldiphénylsilyloxy)-8,11-bis-hydroxy-14-eicosene-9-yne de formule :

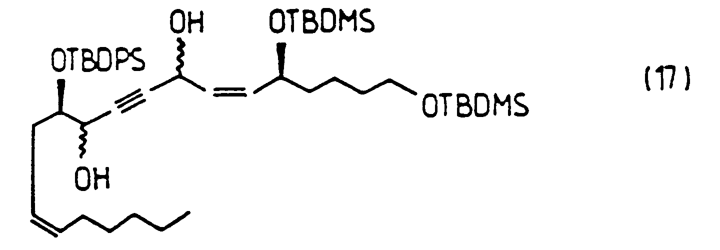

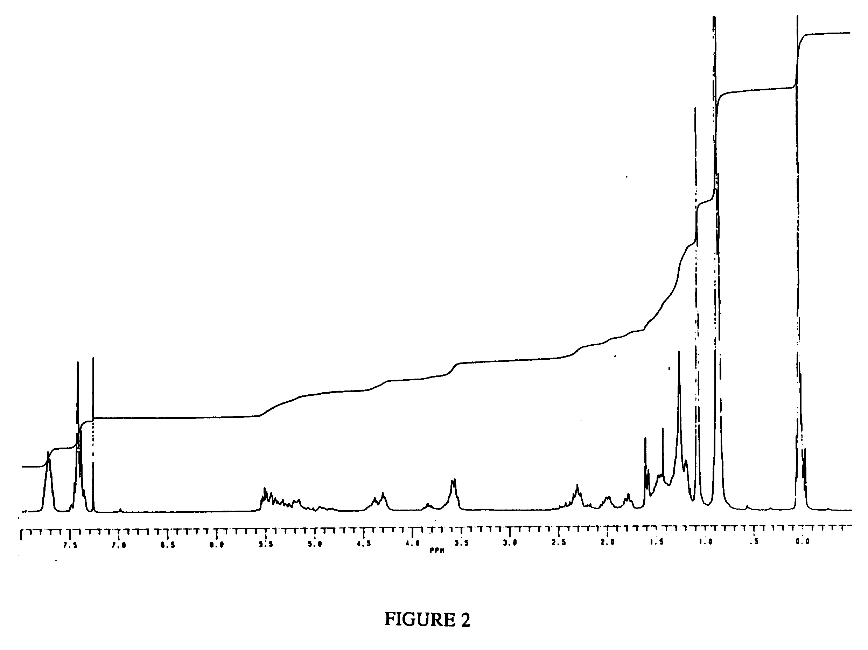

L'alcool propargylique (16) (600 mg, 1,3 mmol., 1 équivalent) dans le THF (5 ml) est traité par une solution 1,5 M de n-butyllithium dans l'hexane (2 ml, 3mmol., 2,25 équivalents) à -78°C. On laisse ensuite remonter la température à -40°C pendant une à deux heures et on ajoute, à cette température, le [4(Z), 2(R)]-2-(t-butyldiphénylsilyloxy)-4-decen-1-al de formule: 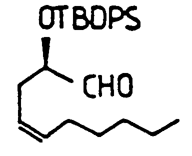 (600 mg, 1,3 mmol., 1 équivalent) en solution dans le THF (5 ml). L'agitation est poursuivie à -40°C pendant une heure puis à température ambiante pendant une heure. Après dilution à l'éther, hydrolyse avec une solution saturée de NH₄Cl, lavage des phases organiques avec une solution saturée de NaCl, évaporation des solvants et purification par chromatographie (éther/hexane : 30/70), on obtient 850 mg (71 %) de diol propargylique (17) (mélange de diastéréoisomères). La figure 2 représente le spectre RMN du proton (CDCl₃, 200 M Hz)
([α]_{D} = +11 (C = 2, CHCl₃)).

### 7ème étape : Préparation de [6(Z), 9(Z), 14(Z), 5(S), 12 (R)]-1,5-bis (t-butyldiméthylsilyloxy)-12-(t-butyldiphénylsilyloxy)-8,11-dihydroxy-6, 9, 14-eicosatriène de formule

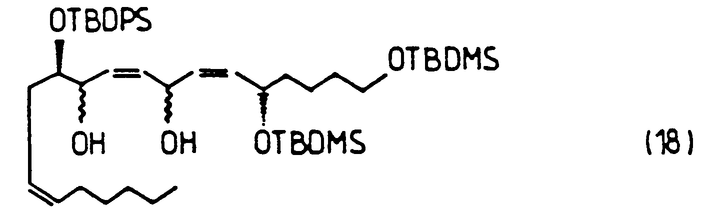

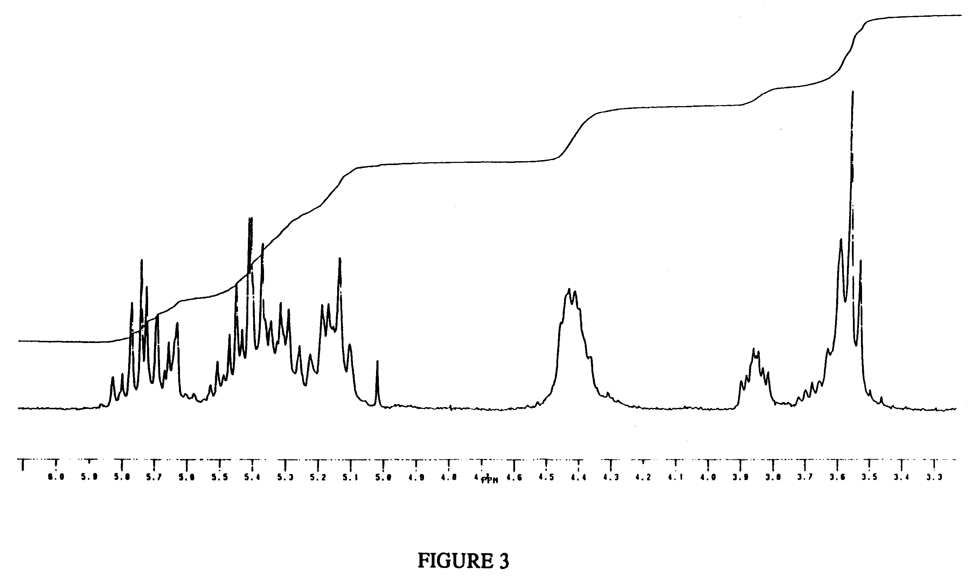

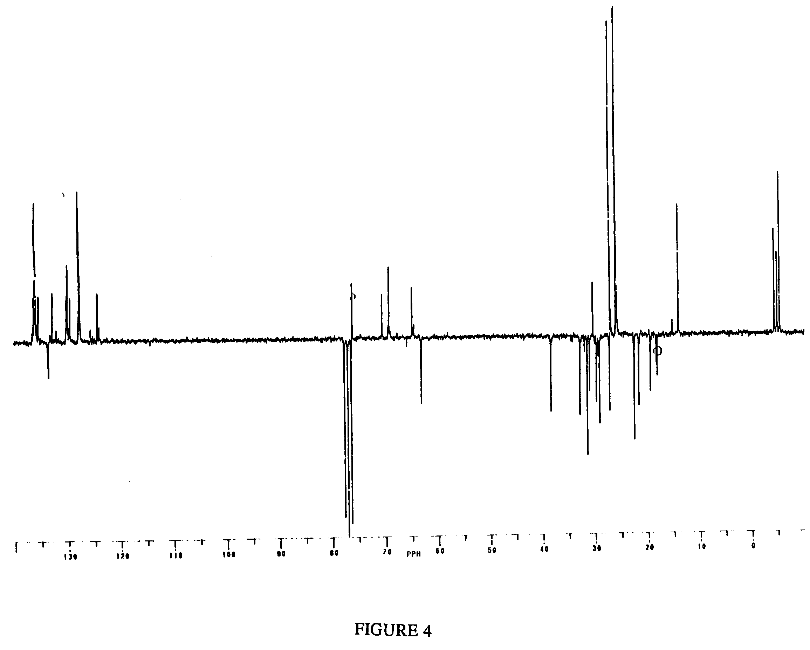

Le diol propargylique (17) (400 mg, 0,5 mmol.) en solution dans l'hexane (40 ml) est réduit par le catalyseur de Lindlar (200 mg) en présence de quinoléine (50 ml) sous atmosphère d'hydrogène pendant 4 heures. Après dilution à l'éther et filtration sur silicagel, les solvants sont évaporés et le produit purifié par chromatographie (éther/hexane : 50/50). On obtient 225 mg (55 %) de diol (18) sous forme de mélange de diastéréoisomères. La figure 3 représente le spectre RMN du proton (CDCl₃, 200 M Hz) et la figure 4 le spectre RMN du 13C (CDCl₃, 200 M Hz)
([α]_{D} = +11 (C = 2, CHCl₃)).

### 8ème étape : Préparation de [6Z, 9Z, 14Z, 5(S), 12(R)]-1,5-bis (t-butyldiméthylsilyloxy)-12-(t-butyldiphénylsilyloxy)-8,11-bis-benzoxy-6, 9, 14-eicosatriène de formule :

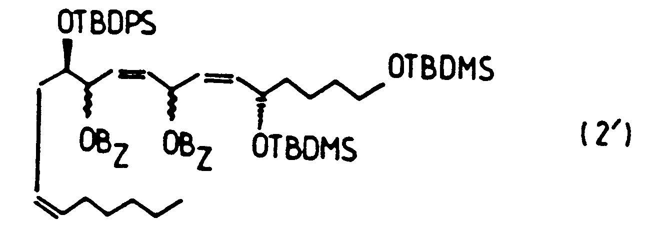

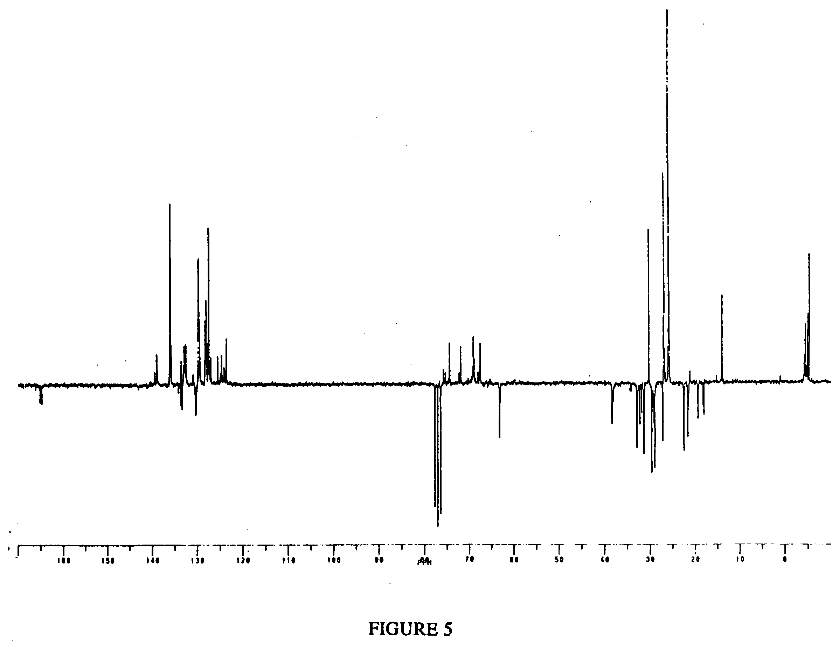

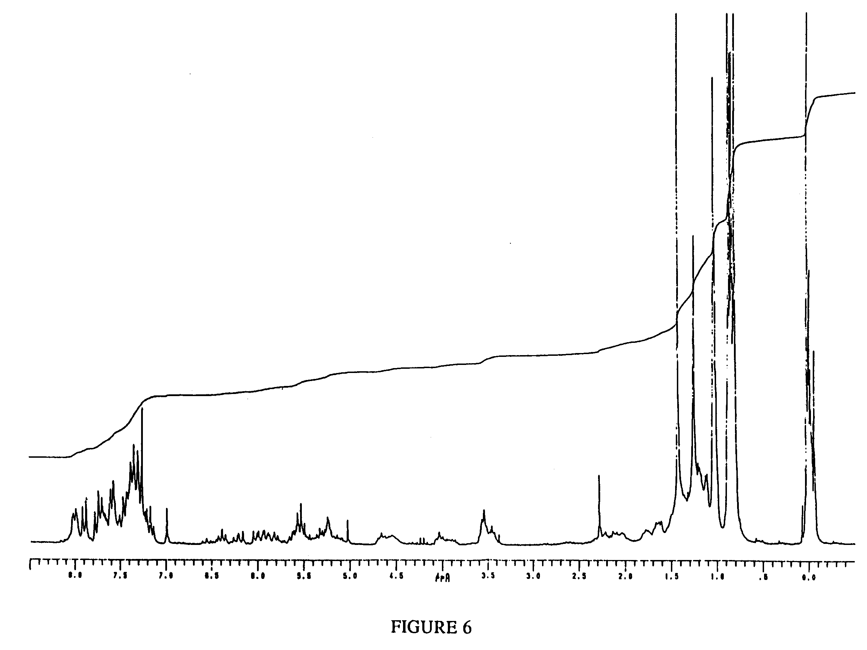

Le diol (18) (205 mg, 0,25 équivalent) en solution dans la pyridine (5 ml) est traité par du chlorure de benzoyle (150 µl, 181,5 mg, 5 équivalents) à 0°C. Après trois heures d'agitation à température ambiante, on dilue avec de l'éther et on ajoute H₂SO₄ à 5 %. La phase organique est lavée par une solution saturée de NH₄Cl et de NaCl. Après évaporation des solvants et purification par chromatographie (ether/hexane : 5/95), on isole 235 mg (90 %) de dibenzoate (2') sous forme de mélange de diastéréoisomères. La figure 5 représente le spectre RMN obtenu du 13C (CDCl₃, 200 M Hz) et la figure 6 le spectre RMN du proton (CDCl₃, 200 M Hz).

### 9ème étape : Préparation de [6Z, 8E, 10E, 14Z, 5(S), 12(R)]-1,5-bis(t-butyldiméthylsilyloxy)-12(t-butyldiphénylsilyloxy)-6, 8, 10, 14-eicosatetraène de formule :

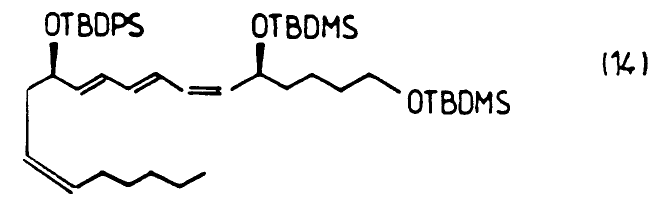

Le dibenzoate (2') (210 mg, 0,2 mmol.) en solution dans un mélange de THF (10 ml) et de méthanol (3 ml) est traité par Na₂HPO₄ (200 mg) et après refroidissement à -20°C, par l'amalgame de sodium à 6 % en poids (1 g, 2,6 mmol., 13 équivalents) préparé selon "Reagents for Organic Synthesis, Fieser and Fieser, John Wiley, N.Y.. 1967, Vol.1, page 1030". Après deux heures de réaction, le mélange réactionnel est dilué à l'éther puis filtré sur gel de silice. Après évaporation des solvants, le produit est purifié par chromatographie. On obtient 130 mg (81 %) de triène (14) pur.

Le produit a les caractéristiques suivantes :
a) [α]_{D} = + 48 (c = 0,3, CHCl₃)
b) ¹H RMN (CDCl₃, 200 MHz) : δ : 7,71 et 7,39 (2m, 10H, arom.H), 6,29 (dd, 1H, J = 11,1 et 14,3 Hz, H-8), 6,08(m, 1H, H-10), 5,94 (t, 1H, J = 10,9 Hz H-7), 5,92 (dd, 1H, J = 10,0 et 15,0 Hz, H-9), 5,69 (dd, 1H, J = 6,8, et 14,7 Hz, H-11), 5,5-5,2 (m, 3H, H-6, H-14 et H-15), 4,55 (m, 1H, H-5), 4,22 (q, 1H, J = 6,8 Hz, H-12), 3,63 (t, 2H, J = 6,1 Hz, H-1), 2,30 (m, 2H, H-16), 1,88 (q, 2H, J = 6,4 Hz, H-13), 1,7-1,2 (m, 12H, H-2, H-3, H-4, H-17, H-18, H-19), 1,11 (s, 9H, t-Bu), 0,95-0,85 (m, 21H, t-Bu, H-20), 0,11, 0,08 et 0,07 (3s, 12H, CH₃Si),
c) ¹³C RMN (CDCl₃) : δ : 136,5, 135,5, 133,4, 132,1, 130,2, 127,5, 127,3 et 124,5 (8 vinyl C), 136 (2C), 135,9 (2C), 134,3, 134,1, 129,5, 129,4, 127,5 (2C) et 127,4 (2C) [10 arom.C], 74 et 69,1 (C-5 et C-12), 63,2 (C-1), 38,3, 36, 32,8, 31,5, 29,2,27,3, 22,6 et 21,8 (8 aliphat. C), 27,1, 26,0 et 25,9 (9C, t-Bu), 19,3, 18,4 et 18,2 (3C, t-Bu), 14,1 (C-20), -4,2, -4,8 et -5,3 (CH₃-Si).

### 10ème étape : Préparation de 5-(t-butyldiméthylsilyloxy)-12-(t-butyldiphénylsilyloxy)-LTB₄ de formule :

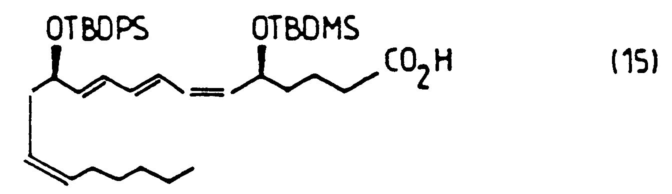

Le triène (14) (100 mg, 0,12 mmol.) en solution dans le diméthylformamide est traité par le pyridinium dichromate (PDC) (450 mg, 1,2 mmol., 10 équivalents) à température ambiante pendant 24 heures. On rajoute à nouveau du PDC (225 mg, 0,6 mmol., 5 équivalents) et on agite à nouveau pendant 24 heures. Après dilution avec de l'éther, on traite par une solution saturée de NH₄Cl. La phase organique est ensuite lavée par une solution saturée de NaCl. Après évaporation des solvants et chromatographie préparative sur couche mince (silice prélavée avec de l'éthanol, éluent : éther/hexane : 30/70 déoxygéné), on isole 53 mg (62 %) de l'acide (15).

Le produit obtenu a les caractéristiques suivantes :
a) [α]_{D} = + 65 (c = 0,6, CHCl₃)
b) ¹H RMN (CDCl₃, 200 MHz) : δ: 7,65 et 7,35 (2m, 10H, arom.H), 6,25 (dd, 1H, J = 11,0 et 14,3 Hz, H-8) 6,06 (m, 1H, H-10), 5,33 (t, 1H, J = 11,0 Hz, H-7), 5,91 (dd, 1H, J = 9,8 et 15,0 Hz, H-9), 5,66 (dd, 1H, J = 6,7 et 14,7 Hz, H-11), 5,5-5,2 (m, 3H, H-6, H-14, H-15), 4,53 (m, 1H, H-5), 4,20 (q, 1H, J = 6,7 Hz, H-12), 2,4-2,1 (m, 4H, H-2, H-16), 1,85 (m, 2H, H-13), 1,7-1,2 (m, 10H, H-3, H4, H-17, H-18, H-19), 1,08 (s, 9H,t-Bu), 0,9-0,8 (m, 12H, t-Bu et H-20), 0,08 et 0,04 (2s, CH₃-Si),
c) ¹³C RMN (CDCl₃) : δ : 179,4 (C-1), 136,8, 134,8, 134,7, 132,1, 130,1 127,9, 127,0 et 124,5 (8 vinyl C), 136,0 (2C), 135,9 (2C), 134,3, 134,1, 129,5, 129,4, 127,5 (2C) et 127,4 (2C) [12 arom. C], 73,9 et 68,6 (C-5, C-12) 37,7, 36,0, 33,9, 31,5, 29,2, 27,3, 22,6 et 20,6 (8 aliphat. C), 27,0 et 25,9 (6 C, t-Bu),19,3 et 18,1 (2C, t-Bu), 14,1 (C-20), -4,2, et -4,8 (CH₃-Si).

### 11ème étape : Préparation du LTB₄ de formule :

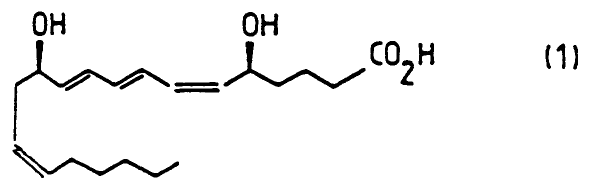

L'hydrolyse des éthers silylés de l'acide (15) a été faite dans les conditions décrites par Kobayashi et coll. J. Org. Chem. 1990 55, 5324. On ajoute une solution 1,1M de fluorure de tétrabutylammonium (TBAF) dans le THF (650 µl, 0,7 mmol.) à l'acide (15) (50 mg, 0,07 mmole) dissous dans le THF (2 ml). Après 24 heures, à température ambiante, on dilue avec de l'éther, on hydrolyse avec un tampon phosphate (préparé à partir de 7,38 g de Na₂HPO₄, 12H₂O, 2,04 g d'acide citrique dans 200 ml d'eau). On lave la phase organique avec une solution saturée de NaCl, on évapore les solvants et on purifie par chromatographie sur plaque de silice préparative (prélavée à l'éthanol, éluent : méthanol/éther : 2/98 déoxygéné) pour obtenir 16,7 mg (71 %) de LTB₄.

Le LTB₄ obtenu a les caractéristiques suivantes :
a) [α]_{D} = + 12,7 (c = 0,3, CDCl₃)
b) ¹H RMN (CDCl₃, 200 MHz) : δ : 6,49 (dd, 1H, J = 11,7 Hz et 14,0 Hz, H-8), 6,27 (m, 2H, H-9 et H-10), 6,08 (t, 1H, J = 11,0 Hz, H-7), 5,78 (dd, 1H, J = 6,2 et 14,4 Hz, H-11), 5,65-5,30 (m, 3H, H-6, H-14 et H-15), 4,60 (m, 1H, H-5), 4,22 (q, 1H, J = 6,4 Hz, H-12), 2,5-2,2 (m, 4H, H-2 et H-13), 2,15-1,95 (m, 2H, H-16), 1,8-1,2 (m, 12H, H-3, H-4, H-17, H-18, H-19, H-20), 0,88 (t, 3H, J = 6,5 Hz, H-20)
c) ¹³C RMN (CDCl₃) : 178,9 (C-1), 137,1, 134,8, 134,1, 132,9, 130,4, 127,7, 127,0, 124,4 (8 vinyl C), 72,9 et 68,1 (C-5 et C-12), 36,9, 35,6, 33,9, 31, 5, 29,2, 27,3, 22,6, 20,5 (8 aliphat., C), 14,1 (C-20).

Le spectre RMN du LTB₄ obtenu correspond à celui décrit dans la littérature pour ce composé.

## Revendications

1. Procédé de synthèse stéréospécifique de leucotriène B₄ (LTB₄) de configuration 6Z, 8E, 10E ayant pour formule 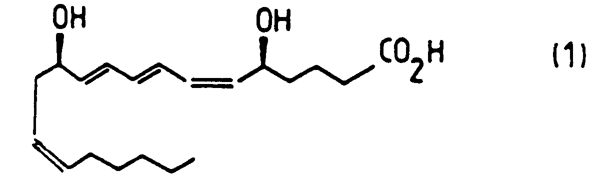 caractérisé par le fait que :
1) on prépare un dibenzoate de formule (2) 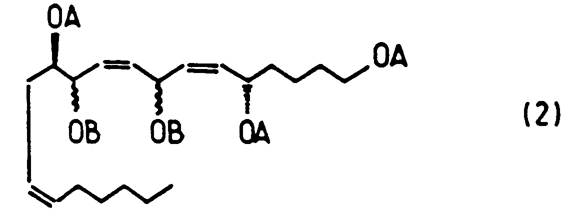 formule (2) dans laquelle A représente un groupe silyle, et B représente un radical benzoyle non substitué ou benzoyle substitué par un radical alkyle ou alcoxy en C₁-C₆ ;
2) on fait subir au dibenzoate de formule (2) une élimination réductrice contrôlée à l'aide d'un amalgame de sodium à une température comprise entre -10°C et -30°C, en présence d'un solvant, de façon à obtenir un triéther de formule (3): 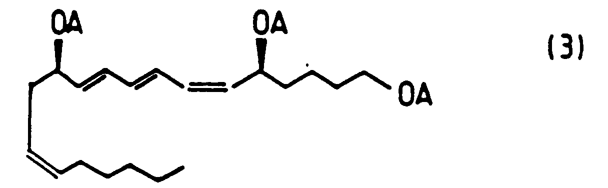 dont on transforme, ensuite, les groupes -OA en positions 5 et 12 en groupe -OH et le groupe -CH₂OA en position 1 en groupe -COOH pour obtenir le leucotriène B₄ de formule (1).

2. Procédé selon la revendication 1, caractérisé par le fait que, dans la formule (2), A représente un groupe silyle choisi dans le groupe formé par le tertiobutyldiméthylsilyle (TBDMS) et le tertiobutyldiphénylsilyle (TBDPS), les groupes A pouvant être différents l'un de l'autre.

3. Procédé selon la revendication 2, caractérisé par le fait que le dibenzoate de formule (2) a pour formule 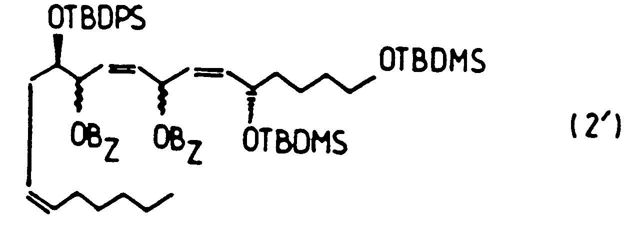 Bz étant un radical benzoyle non substitué.

4. Procédé selon l'une des revendications 1 à 3, caractérisé par le fait qu'on effectue l'élimination réductrice à l'aide d'un amalgame de sodium en présence d'un solvant choisi dans le groupe formé par le tétrahydrofuranne, le méthanol ou leurs mélanges.

5. Procédé selon l'une des revendications 1 à 4, caractérisé par le fait qu'on transforme le triéther de formule (3) en leucotriène B₄ de formule (1) par les étapes suivantes :
1) dans une première étape, on fait réagir le triéther de formule (3) avec le dichromate de pyridinium à température ambiante en présence d'un solvant, de façon à obtenir l'acide de formule (4): 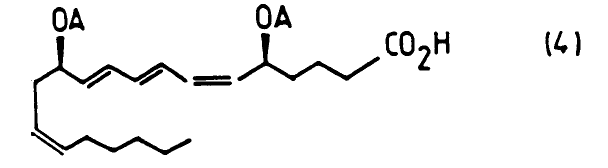 et
2) dans une seconde étape, on traite l'acide de formule (4) par une solution de fluorure de tétrabutylammonium dans un solvant pour obtenir le leucotriène B₄ de formule (1).

6. Procédé selon la revendication 5, caractérisé par le fait qu'à la première étape, on utilise comme solvant le diméthylformamide et à la seconde étape, on utilise comme solvant le tétrahydrofuranne.

7. Procédé selon l'une des revendications 1 à 6, caractérisé par le fait qu'on prépare le dibenzoate de formule (2) par les étapes suivantes :
1) dans une première étape, on fait réagir un alcool propargylique de formule (5) : 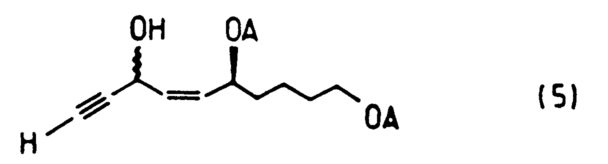 et un aldéhyde de formule (6): 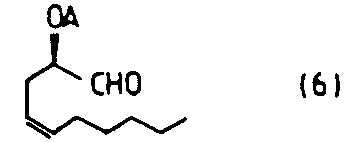 l'alcool propargylique de formule (5) étant préalablement traité par le n-butyllithium, à une température comprise entre -70°C et -90°C, dans un solvant, puis réagissant avec l'aldéhyde de formule (6) dissous dans un solvant, la température étant progressivement ramenée à la température ambiante, de façon à obtenir le diol propargylique de formule (7): 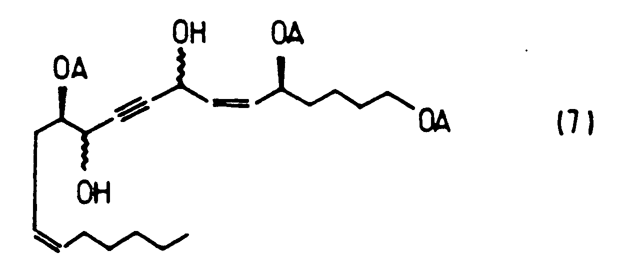
2) dans une deuxième étape, on réduit le diol propargylique de formule (7) en solution dans un solvant avec le catalyseur de Lindlar en présence de quinoléine à température ambiante, de façon à obtenir le diol de formule (8): 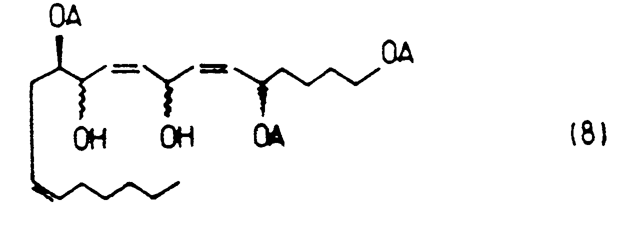
3) dans une troisième étape, le diol de formule (8) est traité par le chlorure de benzoyle en solution dans un solvant à température ambiante de façon à obtenir le dibenzoate de formule (2).

8. Procédé selon la revendication 7, caractérisé par le fait qu'on utilise comme solvant, dans la première étape, le tétrahydrofuranne et comme solvant, dans la deuxième étape, l'hexane.

9. Procédé selon l'une des revendications 7 ou 8, caractérisé par le fait qu'on prépare l'alcool propargylique de formule (5): 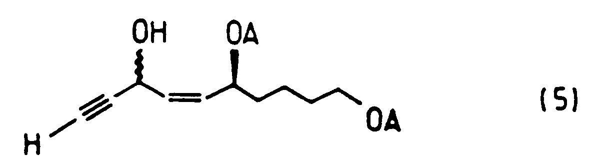 par les étapes suivantes :
1) dans une première étape, on utilise, comme produit de départ, l'aldéhyde de formule (9): 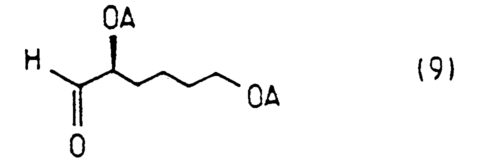 et on le fait réagir avec de la triphénylphosphine et du zinc en poudre dans un solvant, puis avec CBr₄, à température ambiante, de façon à obtenir le dibromure de formule (10): 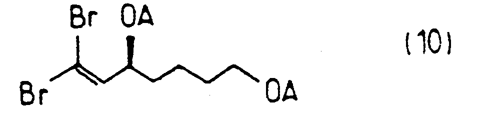
2) dans un deuxième stade, on fait réagir le dibromure de formule (10) avec du n-butyllithium à une température inférieure à -50°C dans un solvant pour obtenir l'alcyne de formule (11): 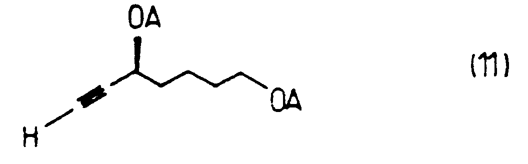
3) dans une troisième étape, on fait réagir l'alcyne de formule (11) en solution dans un solvant avec une solution de n-butyllithium à une température comprise entre -70°C et -90°C puis avec une solution de N-formylpipéridine, la température étant ramenée progressivement à la température ambiante, de façon à obtenir l'aldéhyde propargylique de formule (12): 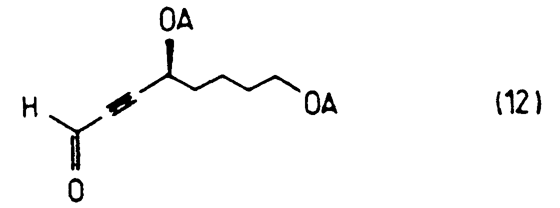
4) dans une quatrième étape, on met l'aldéhyde de formule (12) en présence du catalyseur de Lindlar et de quinoléine en milieu solvant sous atmosphère d'hydrogène, de façon à obtenir l'aldéhyde de formule (13): 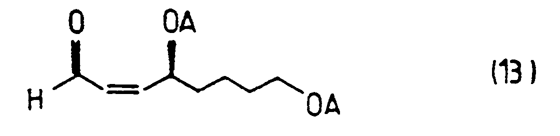 et
5) dans une cinquième étape, on dissout l'aldéhyde de formule (13) dans un solvant puis on ajoute du bromure d'acétylène-magnésium de façon à obtenir l'alcool propargylique de formule (5).

10. Procédé selon la revendication 9, caractérisé par le fait que:
- à la première étape, on utilise le dichlorométhane comme solvant ;
- à la deuxième étape, on effectue la réaction à une température de l'ordre de -80°C dans le tétrahydrofuranne ;
- à la troisième étape, on fait réagir l'alcyne de formule (11) en solution dans le tétrahydrofuranne ;
- à la quatrième étape, on utilise le tétrahydrofuranne comme solvant, et
- à la cinquième étape, on dissout l'aldéhyde de formule (13) dans le tétrahydrofuranne.

11. Dibenzoate de formule 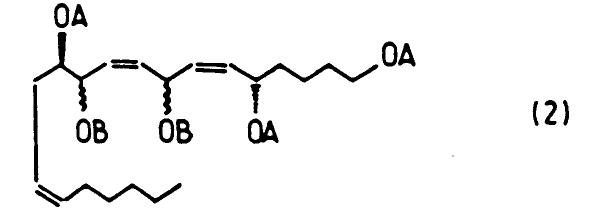 formule dans laquelle A représente, indépendamment l'un de l'autre un groupe tertiobutyldiméthylsilyle (TBDMS) ou un groupe tertiobutyldiphénylsilyle (TBDPS) et B représente un radical benzoyle non-substitué (Bz) ou un radical benzoyle substitué par un radical alkyle ou alkoxy en C₁-C₆.

12. Dibenzoate selon la revendication 11, de formule 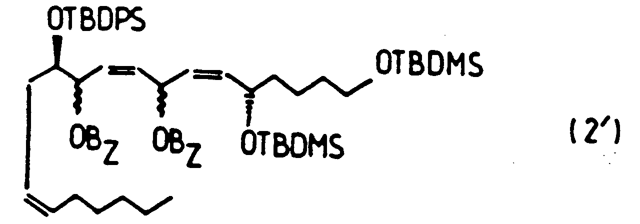 où TBDMS, TBDPS et Bz ont la signification donnée dans la revendication 11.

13. Triène de formule 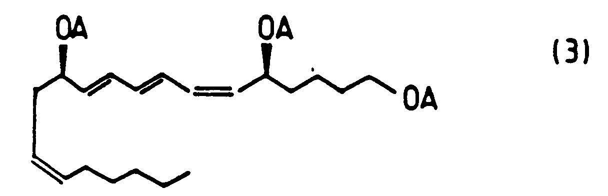 où A a la même signification que dans la revendication 11.

14. Triène selon la revendication 13, de formule 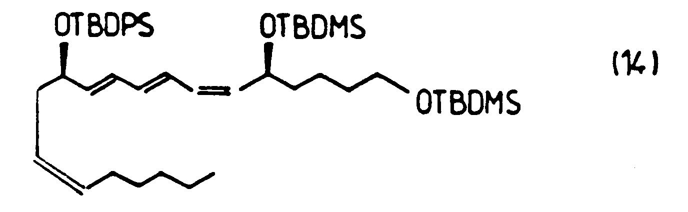 où TBDMS et TBDPS ont la même signification que dans la revendication 11.

15. Acide de formule 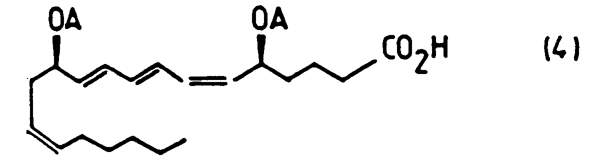 où A a la même signification que dans la revendication 11.

16. Acide selon la revendication 15, de formule 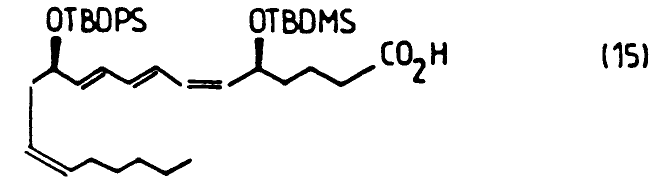 où TBDMS et TBDPS ont la même signification que dans la revendication 11.

17. Alcool propargylique de formule 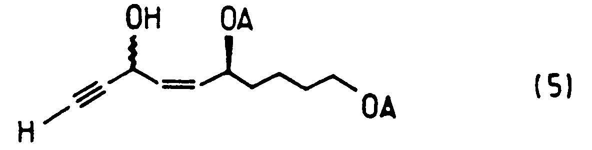 où A a la même signification que dans la revendication 11.

18. Alcool propargylique selon la revendication 17 de formule 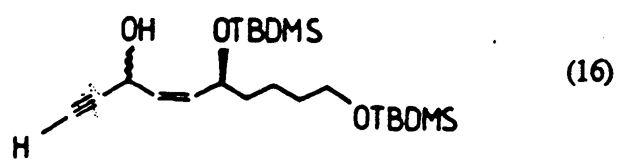 où TBDMS a la même signification que dans la revendication 11.

19. Diol propargylique de formule 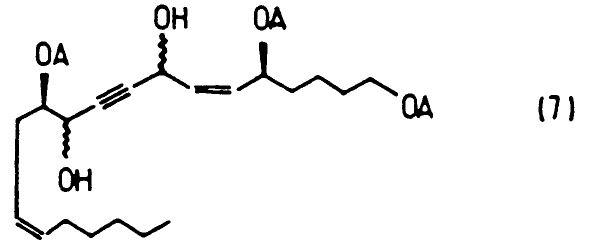 où A a la même signification que dans la revendication 11.

20. Diol propargylique selon la revendication 19, de formule 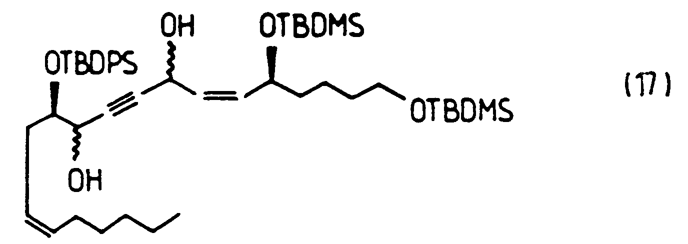 où TBDMS et TBDPS ont la même signification que dans la revendication 11.

21. Dibromure de formule 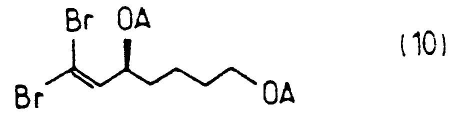 où A a la même signification que dans la revendication 11.

22. Dibromure selon la revendication 21, de formule 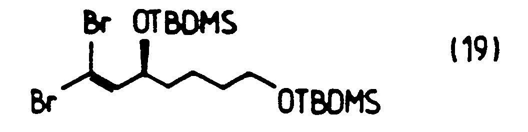 où TBDMS a la même signification que dans la revendication 11.

23. Alcyne de formule 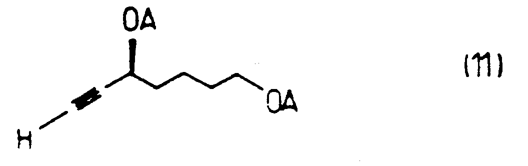 où A a la même signification que dans la revendication 11.

24. Alcyne selon la revendication 23, de formule 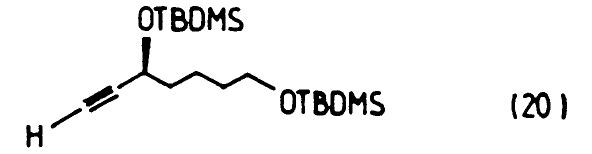 où TBDMS a la même signification que dans la revendication 11.

25. Aldéhyde propargylique de formule 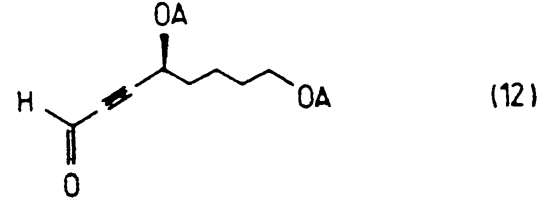 où A a la même signification que dans la revendication 11.

26. Aldéhyde propargylique selon la revendication 25, de formule 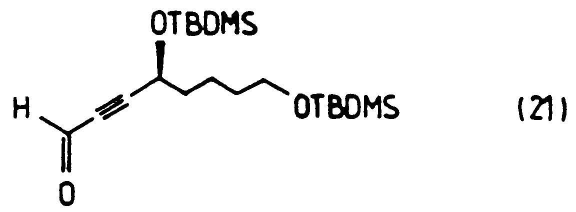 où TBDMS a la même signification que dans la revendication 11.

27. Aldéhyde de formule 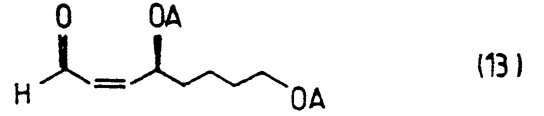 où A a la même signification que dans la revendication 11.

28. Aldéhyde selon la revendication 27, de formule 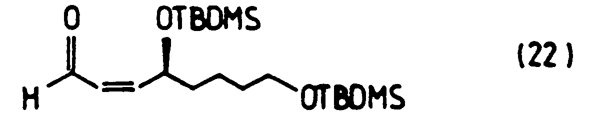 où TBDMS a la même signification que dans la revendication 11.

29. Aldéhyde de formule 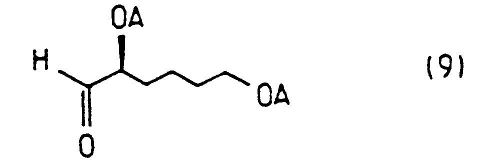 où l'un au moins des deux groupes A représente TBDPS, TBDPS ayant la signification donnée dans la revendication 11.

## Claims

1. Process for the stereospecific synthesis of leukotriene B₄ (LTB₄) of 6Z, 8E, 10E configuration having the formula 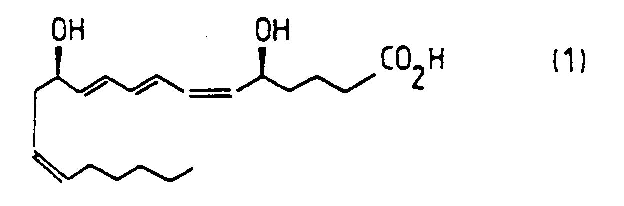 characterized in that:
1) a dibenzoate of the formula (2) 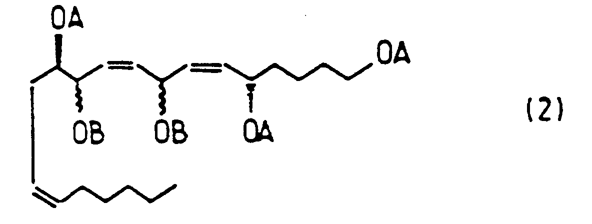 in which formula (2) A represents a silyl group and B represents an unsubstituted benzoyl radical or a benzoyl radical substituted by a C₁-C₆ alkoxy or alkyl radical, is prepared;
2) the dibenzoate of formula 2) is subjected to a controlled reductive elimination using a sodium amalgam at a temperature between -10°C and -30°C, in the presence of a solvent, so as to obtain a triether of formula (3): 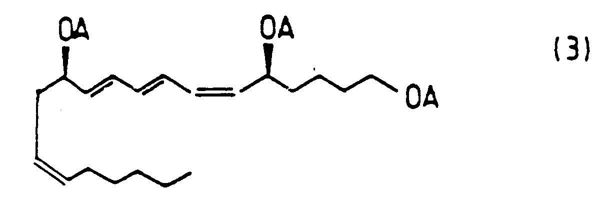 the -OA groups in the 5- and 12-positions of which are then converted to -OH groups and the -CH₂OA group in the 1-position of which is then converted to the -COOH group in order to obtain the leukotriene B₄ of formula (1).

2. Process according to Claim 1, characterized in that, in the formula (2), A represents a silyl group chosen from the group formed by tert-butyldimethylsilyl (TBDMS) and tert-butyldiphenylsilyl (TBDPS), it being possible for the A groups to be different from one another.

3. Process according to Claim 2, characterized in that the dibenzoate of formula (2) has the formula 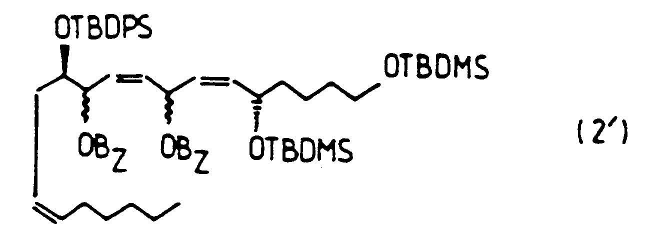 Bz being an unsubstituted benzoyl radical.

4. Process according to one of Claims 1 to 3, characterized in that the reductive elimination is carried out using a sodium amalgam in the presence of a solvent chosen from the group formed by tetrahydrofuran or methanol or their mixtures.

5. Process according to one of Claims 1 to 4, characterized in that the triether of formula (3) is converted to leukotriene B₄ of formula (1) by the following stages:
1) in a first stage, the triether of formula (3) is reacted with pyridinium dichromate at room temperature in the presence of a solvent so as to obtain the acid of formula (4): 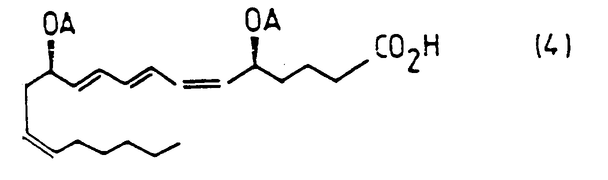 and
2) in a second stage, the acid of formula (4) is treated with a solution of tetrabutylammonium fluoride in a solvent in order to obtain the leukotriene B₄ of formula (1).

6. Process according to Claim 5, characterized in that dimethylformamide is used as solvent in the first stage and tetrahydrofuran is used as solvent in the second stage.

7. Process according to one of Claims 1 to 6, characterized in that the dibenzoate of formula (2) is prepared by the following stages:
1) in a first stage, a propargyl alcohol of formula (5): 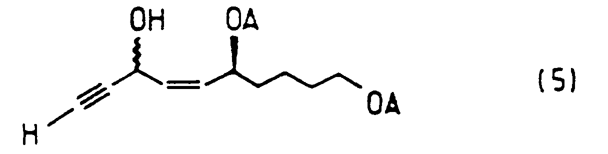 and an aldehyde of formula (6): 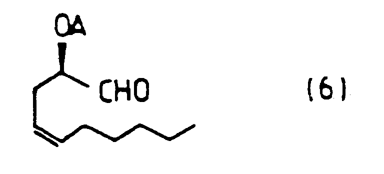 are reacted, the propargyl alcohol of formula (5) being treated beforehand with n-butyllithium, at a temperature between -70°C and -90°C, in a solvent, and then reacting with the aldehyde of formula (6) dissolved in a solvent, the temperature being progressively brought back to room temperature, so as to obtain the propargyl diol of formula (7): 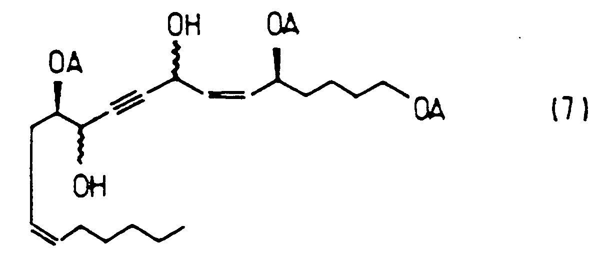
2) in a second stage, the propargyl diol of formula (7), in solution in a solvent, is reduced with the Lindlar catalyst in the presence of quinoline at room temperature, so as to obtain the diol of formula (8): 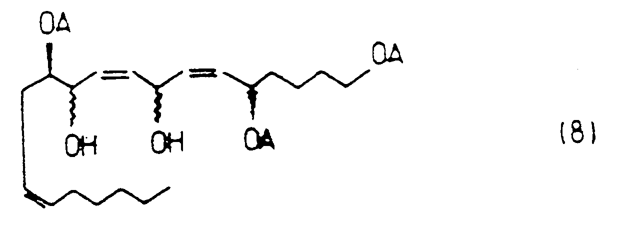 and
3) in a third stage, the diol of formula (8) is treated with benzoyl chloride in solution in a solvent, at room temperature, so as to obtain the dibenzoate of formula (2).

8. Process according to Claim 7, characterized in that tetrahydrofuran is used as solvent in the first stage and hexane is used as solvent in the second stage.

9. Process according to either of Claims 7 and 8, characterized in that the propargyl alcohol of formula (5): 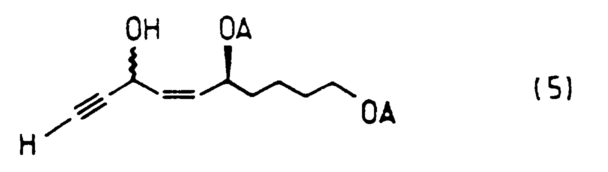 is prepared by the following stages:
1) in a first stage, the aldehyde of formula (9): 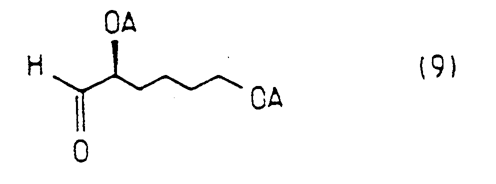 is used as starting material, and it is reacted with triphenylphosphine and zinc powder in a solvent and then with CBr₄, at room temperature, so as to obtain the dibromide of formula (10): 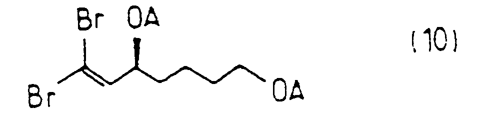
2) in a second stage, the dibromide of formula (10) is then reacted with n-butyllithium at a temperature of less than -50°C in a solvent in order to obtain the alkyne of formula (11): 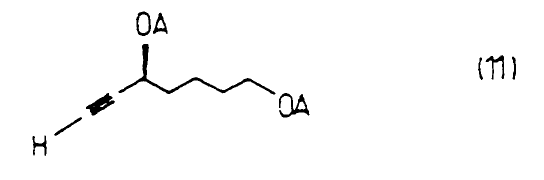
3) in a third stage, the alkyne of formula (11) in solution in a solvent is reacted with an n-butyllithium solution at a temperature of between -70°C and -90°C and then with an N-formylpiperidine solution, the temperature being progressively brought back to room temperature, so as to obtain the propargyl aldehyde of formula (12); 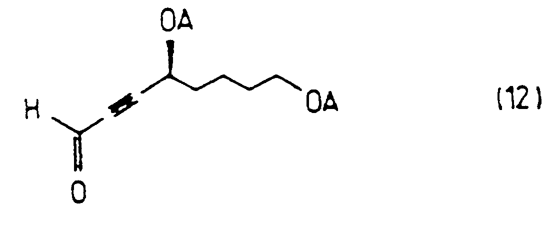
4) in a fourth stage, the aldehyde of formula (12) is brought together with the Lindlar catalyst and with quinoline in solvent medium under a hydrogen atmosphere, so as to obtain the aldehyde of formula (13): 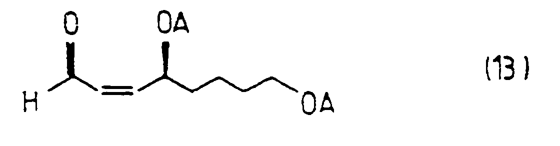 and
5) in a fifth stage, the aldehyde of formula (13) is dissolved in a solvent and then acetylenemagnesium bromide is added so as to obtain the propargyl alcohol of formula (5).

10. Process according to Claim 9, characterized in that:
- in the first stage, dichloromethane is used as solvent;
- in the second stage, the reaction is carried out at a temperature of the order of -80°C in tetrahydrofuran;
- in the third stage, the alkyne of formula (11) is reacted in solution in tetrahydrofuran;
- in the fourth stage, tetrahydrofuran is used as solvent, and
- in the fifth stage, the aldehyde of formula (13) is dissolved in tetrahydrofuran.

11. Dibenzoate of formula 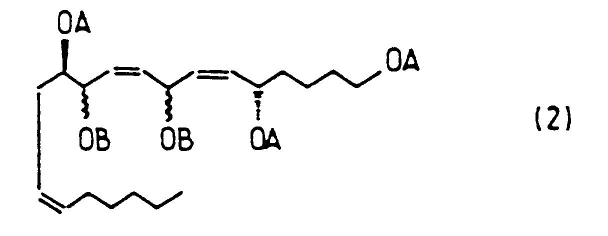 in which formula A represents, independently of one another, a tert-butyldimethylsilyl (TBDMS) group or a tert-butyldiphenylsilyl (TBDPS) group and B represents an unsubstituted benzoyl radical (Bz) or a benzoyl radical substituted by a C₁-C₆ alkoxy or alkyl radical.

12. Dibenzoate according to Claim 11, of formula 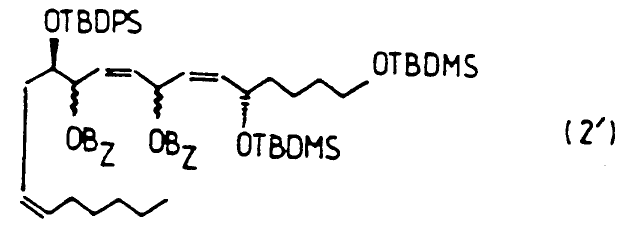 where TBDMS, TBDPS and Bz have the meaning given in Claim 11.

13. Triene of formula 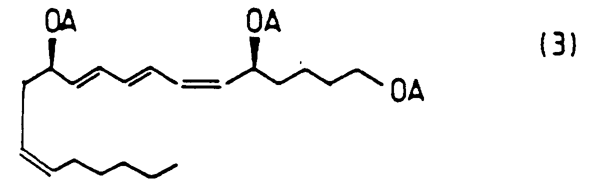 where A has the same meaning as in Claim 11.

14. Triene according to Claim 13, of formula 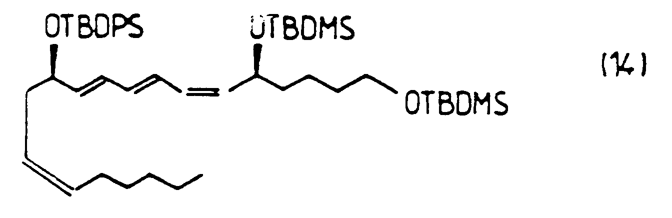 where TBDMS and TBDPS have the same meaning as in Claim 11.

15. Acid of formula 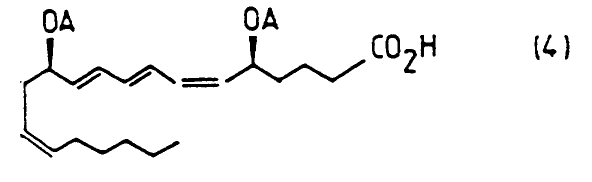 where A has the same meaning as in Claim 11.

16. Acid according to Claim 15, of formula 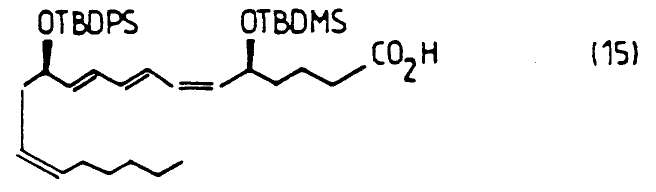 where TBDMS and TBDPS have the same meaning as in Claim 11.

17. Propargyl alcohol of formula 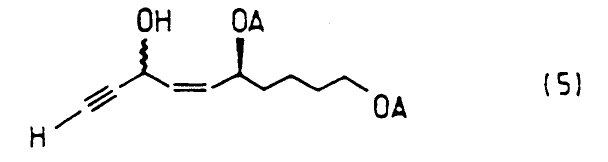 where A has the same meaning as in Claim 11.

18. Propargyl alcohol according to Claim 17, of formula 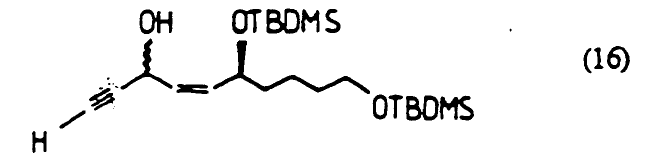 where TBDMS has the same meaning as in Claim 11.

19. Propargyl diol of formula 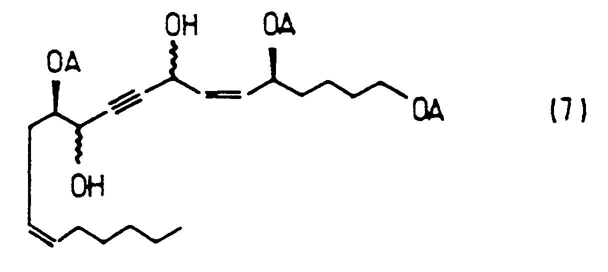 where A has the same meaning as in Claim 11.

20. Propargyl diol according to Claim 19, of formula 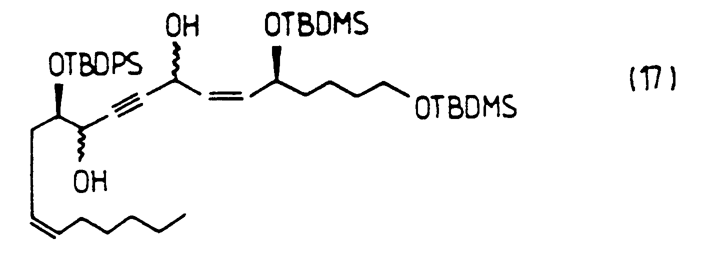 where TBDMS and TBDPS have the same meaning as in Claim 11.

21. Dibromide of formula 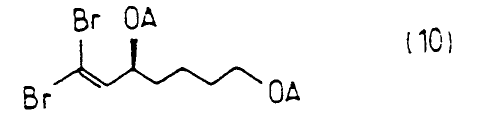 where A has the same meaning as in Claim 11.

22. Dibromide according to Claim 21, of formula 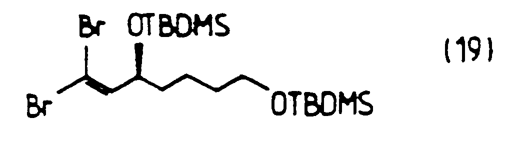 where TBDMS has the same meaning as in Claim 11.

23. Alkyne of formula 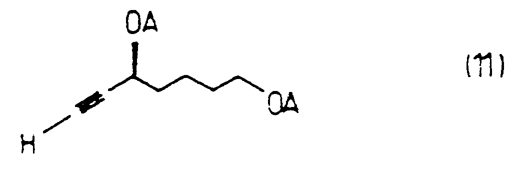 where A has the same meaning as in Claim 11.

24. Alkyne according to Claim 23, of formula 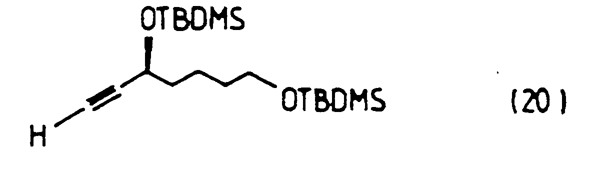 where TBDMS has the same meaning as in Claim 11.

25. Propargyl aldehyde of formula 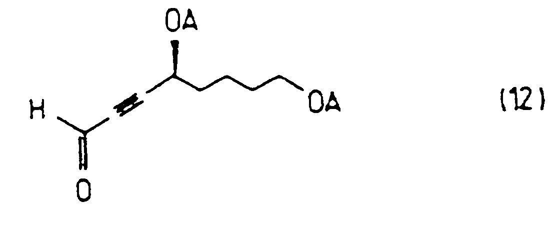 where A has the same meaning as in Claim 11.

26. Propargyl aldehyde according to Claim 25, of formula 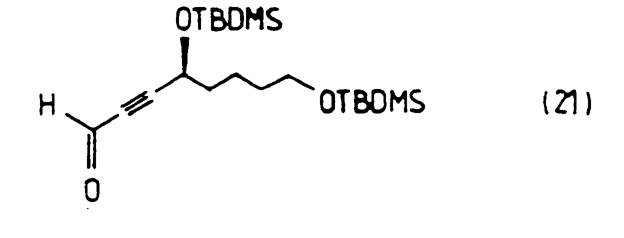 where TBDMS has the same meaning as in Claim 11.

27. Aldehyde of formula 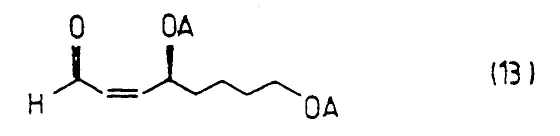 where A has the same meaning as in Claim 11.

28. Aldehyde according to Claim 27, of formula 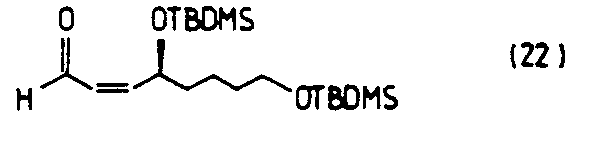 where TBDMS has the same meaning as in Claim 11.

29. Aldehyde of formula 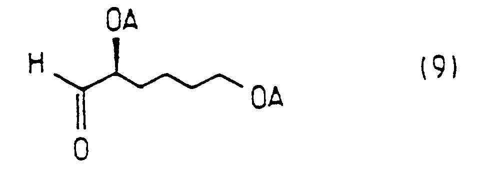 where at least one of the two A groups represents TBDPS, TBDPS having the meaning given in Claim 11.

## Patentansprüche

1. Verfahren zur stereospezifischen Synthese von Leukotrien B₄ (LTB₄) mit 6Z,8E,10E-Konfiguration der Formel 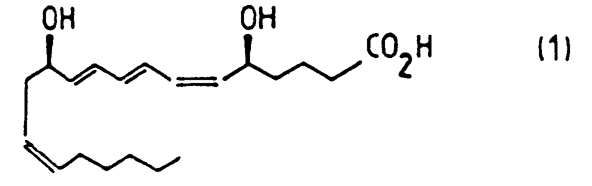 dadurch gekennzeichnet, daß man
1) ein Dibenzoat der Formel (2) 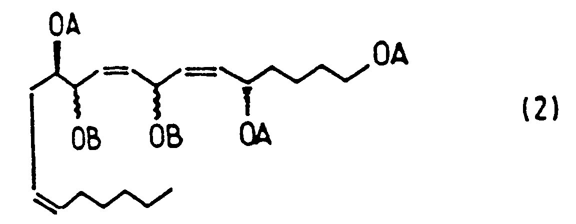 herstellt, worin A für eine Silylgruppe und B für einen unsubstituierten oder mit einem C₁-C₆-Alkyl- oder -Alkoxyrest substituierten Benzoylrest steht;
2) das Dibenzoat der Formel (2) bei einer Temperatur von -10°C bis -30°C und in Gegenwart eines Lösungsmittels einer kontrollierten reduktiven Eliminierung mit Natriumamalgam unterwirft, um einen Triether der Formel (3) 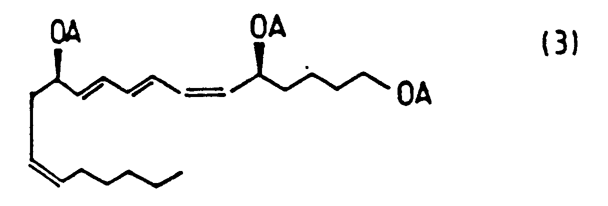 zu erhalten, dessen -OA-Gruppen in 5- und 12-Position man in -OH-Gruppen umwandelt und dessen -CH₂OA-Gruppe in 1-Position man in eine -COOH-Gruppe umwandelt, um das Leukotrien B₄ der Formel (1) zu erhalten.

2. Verfahren nach Anspruch 1, dadurch gekennzeichnet, daß A in der Formel 2 für eine Silylgruppe steht, die ausgewählt ist unter t-Butyldimethylsilyl (TBDMS) und t-Butyldiphenylsilyl (TBDPS), wobei A für voneinander verschiedene Gruppen stehen kann.

3. Verfahren nach Anspruch 2, dadurch gekennzeichnet, daß das Dibenzoat der Formel (2) folgende Formel besitzt 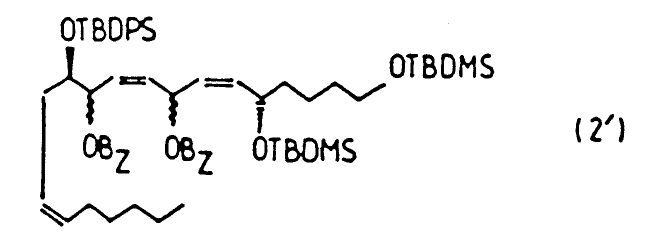 worin Bz für einen unsubstituierten Benzoylrest steht.

4. Verfahren nach einem der Ansprüche 1 bis 3, dadurch gekennzeichnet, daß man die reduktive Eliminierung mit Natriumamalgam in Gegenwart eines Lösungsmittels durchführt, das ausgewählt ist unter Tetrahydrofuran, Methanol oder Gemischen davon.

5. Verfahren nach einem der Ansprüche 1 bis 4, dadurch gekennzeichnet, daß man den Triether der Formel (3) durch folgende Schritte in Leukotrien B₄ der Formel (1) umwandelt:
1) In einer ersten Stufe bringt man den Triether der Formel (3) bei Umgebungstemperatur und in Gegenwart eines Lösungsmittels mit Pyridindichromat zur Reaktion, um eine Säure der Formel (4) 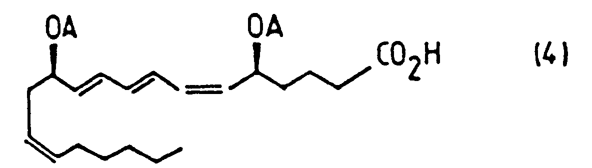 zu erhalten und
2) in einer zweiten Stufe behandelt man die Säure der Formel (4) in einem Lösungsmittel mit einer Tetrabutylammoniumfluorid-Lösung, um das Leukotrien B₄ der Formel (1) zu erhalten.

6. Verfahren nach Anspruch 5, dadurch gekennzeichnet, daß man als Lösungsmittel in der ersten Stufe Dimethylformamid und in der zweiten Stufe Tetrahydrofuran verwendet.

7. Verfahren nach einem der Ansprüche 1 bis 6, dadurch gekennzeichnet, daß man das Dibenzoat der Formel (2) durch folgende Schritte herstellt:
1) In einer ersten Stufe bringt man einen Propargylalkohol der Formel (5) 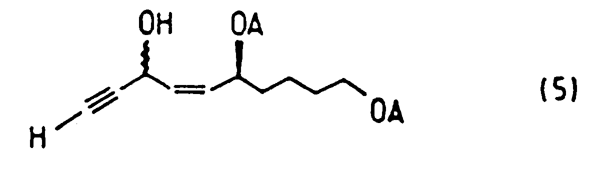 mit einem Aldehyd der Formel (6) 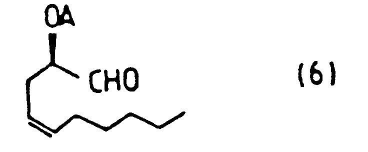 zur Reaktion, wobei der Propargylalkohol der Formel (5) zuvor bei einer Temperatur von -70°C bis -90°C in einem Lösungsmittel mit n-Butyllithium behandelt und dann mit dem in einem Lösungsmittel gelösten Aldehyd der Formel (6) zur Reaktion gebracht wird, wobei die Temperatur allmählich auf Umgebungstemperatur zurückgeführt wird, um ein Propargyldiol der Formel (7) 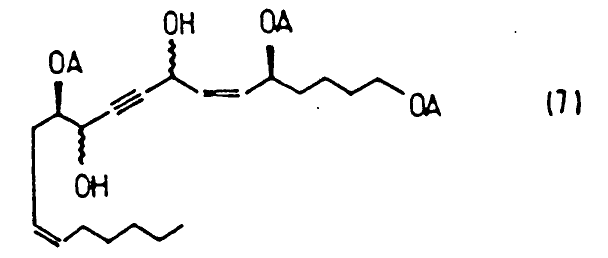 zu erhalten;
2) in einer zweiten Stufe reduziert man das in einem Lösungsmittel gelöste Propargyldiol der Formel (7) in Gegenwart von Chinolin und bei Umgebungstemperatur mit einem Lindlar-Katalysator, um ein Diol der Formel (8) 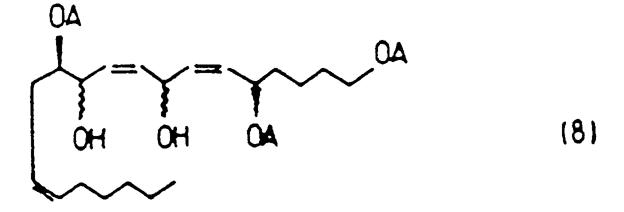 zu erhalten;
3) in einer dritten Stufe wird das Diol der Formel (8) bei Umgebungstemperatur mit in einem Lösungsmittel gelöstem Benzoylchlorid behandelt, um das Dibenzoat der Formel (2) zu erhalten.

8. Verfahren nach Anspruch 7, dadurch gekennzeichnet, daß man als Lösungsmittel in der ersten Stufe Tetrahydrofuran und in der zweiten Stufe Hexan verwendet.

9. Verfahren nach einem der Ansprüche 7 oder 8, dadurch gekennzeichnet, daß man den Propargylalkohol der Formel (5) 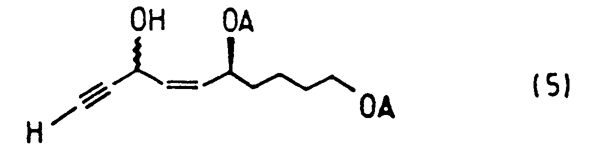 durch folgende Schritte herstellt:
1) in einer ersten Stufe verwendet man als Ausgangsprodukt den Aldehyd der Formel (9) 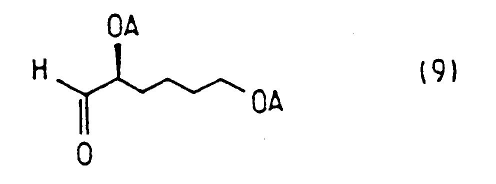 und bringt diesen zunächst mit Triphenylphosphin und mit Zinkpulver in einem Lösungsmittel und anschließend mit CBr₄ bei Umgebungstemperatur zur Reaktion, um das Dibromid der Formel (10) 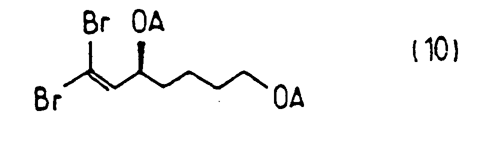 zu erhalten;
2) in einer zweiten Stufe bringt man das Dibromid der Formel (10) mit n-Butyllithium bei einer Temperatur von weniger als -50°C in einem Lösungsmittel zur Reaktion, um das Alkin der Formel (11) 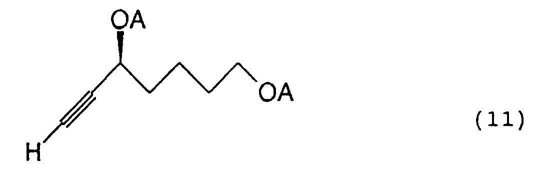 zu erhalten;
3) in einer dritten Stufe bringt man das in einem Lösungsmittel gelöste Alkin der Formel (11) bei einer Temperatur von -70°C bis -90°C mit einer n-Butyllithiumlösung und dann mit einer N-Formylpiperidinlösung zur Reaktion, wobei die Temperatur allmählich auf Umgebungstemperatur zurückgeführt wird, um den Propargylaldehyd der Formel (12) 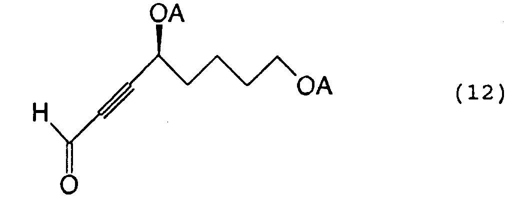 zu erhalten;
4) in einer vierten Stufe gibt man den Aldehyd der Formel (12) in Gegenwart eines Lindlar-Katalysators und von Chinolin in einer Wasserstoffatmosphäre in ein Lösungsmittelmedium, um so den Aldehyd der Formel (13) 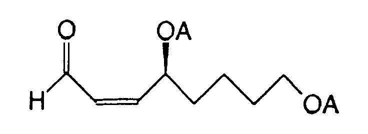 zu erhalten; und
5) in einer fünften Stufe löst man den Aldehyd der Formel (13) in einem Lösungsmittel und gibt dann Acetylenmagnesiumbromid zu, um den Propargylalkohol der Formel (5) zu erhalten.

10. Verfahren nach Anspruch 9, dadurch gekennzeichnet, daß man:
- in der ersten Stufe Dichlormethan als Lösungsmittel verwendet;
- in der zweiten Stufe die Reaktion bei einer Temperatur von etwa -80°C in Tetrahydrofuran durchführt;
- in der dritten Stufe das Alkin der Formel (11) in Lösung in Tetrahydrofuran zur Reaktion bringt;
- in der vierten Stufe Tetrahydrofuran als Lösungsmittel verwendet und
- in der fünften Stufe den Aldehyd der Formel (13) in Tetrahydrofuran löst.

11. Dibenzoat der Formel 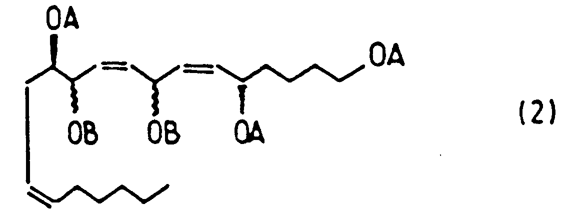 worin die Reste A unabhängig voneinander für eine t-Butyldimethylsilyl (TBDMS)-Gruppe oder eine t-Butyldiphenylsilyl (TBDPS)-Gruppe stehen und B für einen unsubstituierten oder mit einem C₁-C₆-Alkyl- oder - Alkoxyrest substituierten Benzoylrest (Bz) steht.

12. Dibenzoat nach Anspruch 11 der Formel 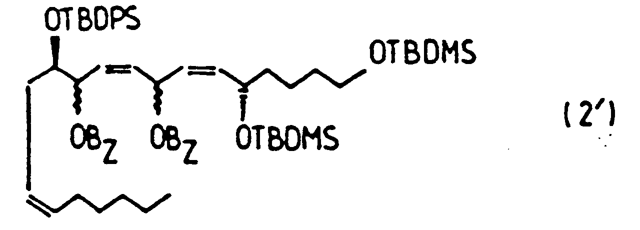 worin TBDMS, TBDPS und Bz die in Anspruch 11 angegebene Bedeutung besitzen.

13. Trien der Formel 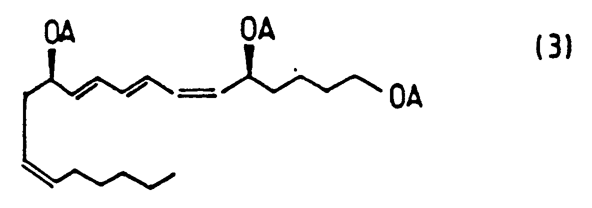 worin A die in Anspruch 11 angegebene Bedeutung besitzt.

14. Trien nach Anspruch 13 der Formel 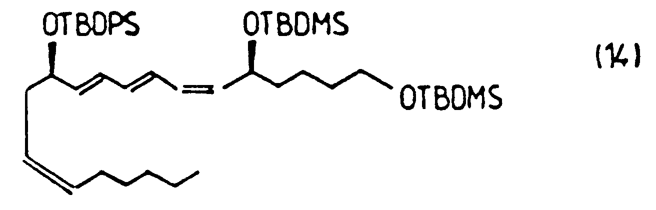 worin TBDMS und TBDPS die in Anspruch 11 angegebene Bedeutung besitzen.

15. Säure der Formel 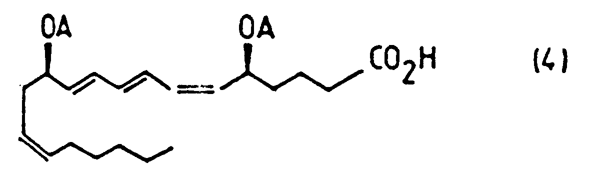 worin A die in Anspruch 11 angegebene Bedeutung besitzt.

16. Säure nach Anspruch 15 der Formel 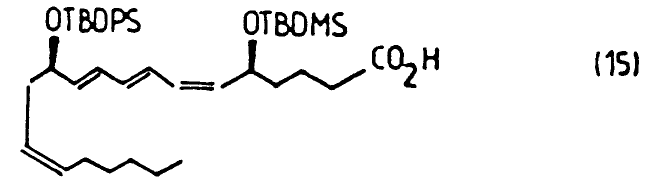 worin TBDMS und TBDPS die in Anspruch 11 angegebene Bedeutung besitzen.

17. Propargylalkohol der Formel 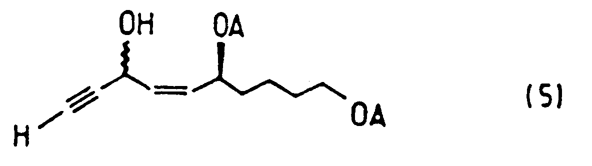 worin A die in Anspruch 11 angegebene Bedeutung besitzt.

18. Propargylalkohol nach Anspruch 17 der Formel 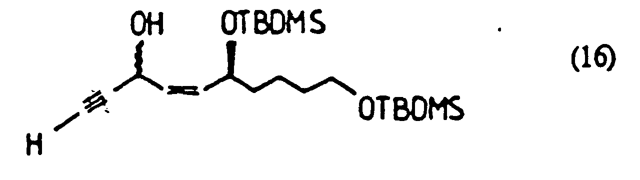 worin TBDMS die in Anspruch 11 angegebene Bedeutung besitzt.

19. Propargyldiol der Formel 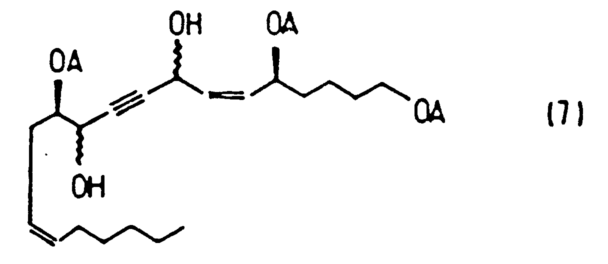 worin A die in Anspruch 11 angegebene Bedeutung besitzt.

20. Propargyldiol nach Anspruch 19 der Formel 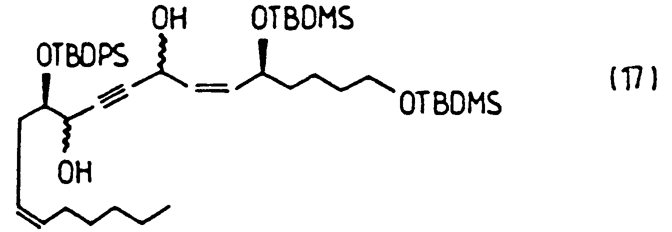 worin TBDMS und TBDPS die in Anspruch 11 angegebene Bedeutung besitzen.

21. Dibromid der Formel 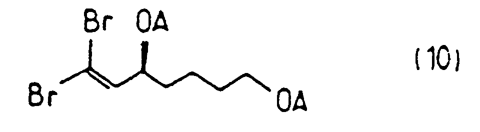 worin A die in Anspruch 11 angegebene Bedeutung besitzt.

22. Dibromid nach Anspruch 21 der Formel 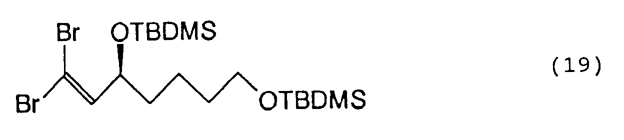 worin TBDMS die in Anspruch 11 angegebene Bedeutung besitzt.

23. Alkin der Formel 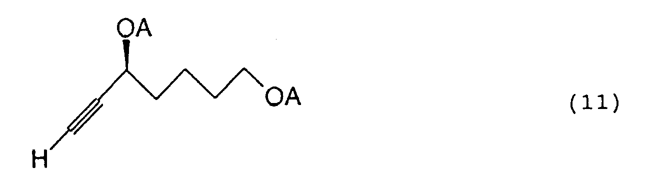 worin A die in Anspruch 11 angegebene Bedeutung besitzt.

24. Alkin nach Anspruch 23 der Formel 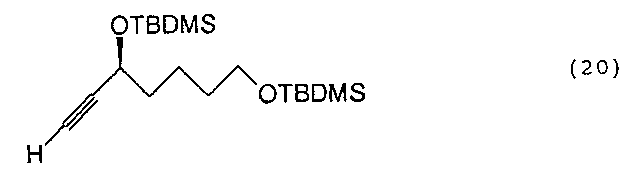 worin TBDMS die in Anspruch 11 angegebene Bedeutung besitzt.

25. Propargylaldehyd der Formel 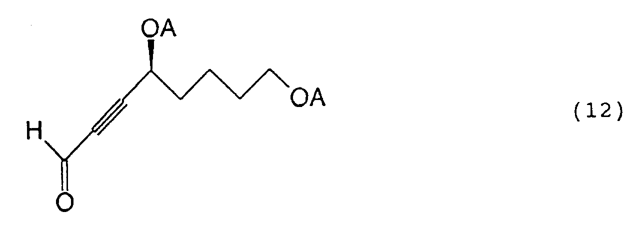 worin A die in Anspruch 11 angegebene Bedeutung besitzt.

26. Propargylaldehyd nach Anspruch 25 der Formel 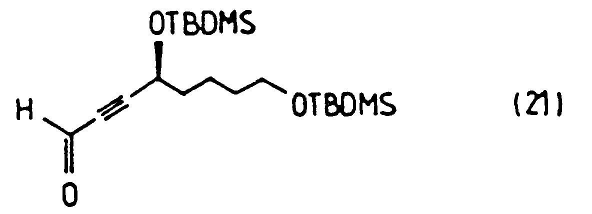 worin TBDMS die in Anspruch 11 angegebene Bedeutung besitzt.

27. Aldehyd der Formel 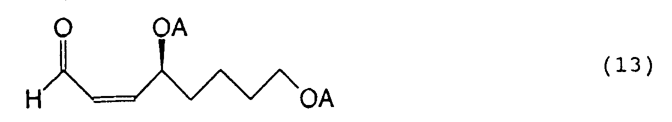 worin A die in Anspruch 11 angegebene Bedeutung besitzt.

28. Aldehyd nach Anspruch 27 der Formel 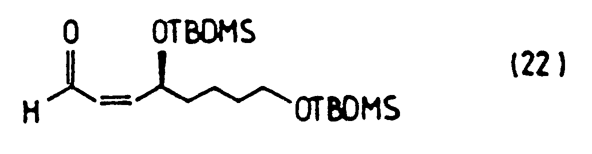 worin TBDMS die in Anspruch 11 angegebene Bedeutung besitzt.

29. Aldehyd der Formel 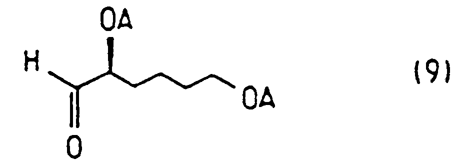 worin wenigstens eine der beiden Gruppen A für TBDPS steht, wobei TBDPS die in Anspruch 11 angegebene Bedeutung besitzt.
